# EUROPEAN PATENT SPECIFICATION

(11) **EP 1 849 472 B1**
(45) Date of publication and mention of the grant of the patent: **10.08.2016**
(21) Application number: 06712410.7
(22) Date of filing: 26.01.2006
(51) Int. Cl.: A61K 8/19, A61K 33/00, A61K 8/73, A61K 8/02, A61K 47/04, A61K 47/36, A61Q 19/00, A61P 17/02

(54) **COMPOSITION FOR PREPARING CARBON DIOXIDE PREPARATION FOR EXTERNAL USE**
ZUSAMMENSETZUNG ZUR HERSTELLUNG EINER KOHLENDIOXIDPRÄPARATION ZUR ÄUSSEREN ANWENDUNG
COMPOSITION POUR PREPARER UNE PREPARATION DE DIOXYDE DE CARBONE A USAGE EXTERNE

(30) Priority: 28.01.2005 JP 2005022094
(43) Date of publication of application: 31.10.2007
(73) Proprietor: Neochemir, Inc., Kobe-shi, Hyogo 651-0087 (JP)
(72) Inventor: TANAKA, Masaya, Hyogo, 6540036 (JP); KIGUCHI, Yasuko, Chuo-ku Kobe-shi Hyogo, 6510087 (JP); DAIKYO, Toshiya, Chuo-ku Kobe-shi Hyogo, 6510087 (JP); OKAMOTO, Mitsuyo, Chuo-ku Kobe-shi Hyogo, 6510087 (JP)
(74) Representative: Sajda, Wolf E.
(86) International application number: PCT/JP2006/301228
(87) International publication number: WO 2006/080398

(56) References cited:
- WO-A1-99/24043
- WO-A1-02/080941
- JP-A- 08 091 817
- JP-A- 60 215 606
- JP-A- 62 077 312
- JP-A- 2002 128 636
- JP-A- 2003 512 894

## Description

### TECHNICAL FIELD

The present invention relates to a composition for preparing a carbon dioxide external preparation with aesthetic and medical effects as defined in the claims.

### BACKGROUND ART

From old times in Europe, it is known that carbon dioxide encourages blood circulation in skins, subcutaneous tissues and/or muscles and activates metabolism after transdermal or transmucosal absorption as a result of studying carbonated spring and the like (See e.g., Isao Nagakura; Carbon Dioxide, Life-Supporting Marvelous Substance, published by Asahi Shimbun, 1988).

Also in Japan, it is reported that carbon dioxide contained in an artificial carbonated spring is effective for the treatment of pressure sores (See e.g., Toshinori Hiyoshi; About Treatment of Pressure Sores Using Bath Preparation of Artificial Carbonated Spring; Comprehensive Rehabilitation 17(8): 605-609, 1989). Under such circumstances, developments of a carbon dioxide external preparation which utilizes remarkable effects of carbon dioxide or a composition for preparing such preparation are further activated in the cosmetic field as well as in the medical field in recent years.

Examples of a composition for preparing a carbon dioxide external preparation described above include a composition for preparing a carbon dioxide external preparation comprising carbon dioxide bubbles in water-containing viscous composition including one or more thickener(s) (WO 99/024043) and a composition comprising water, a thickener and carbon dioxide bubbles which enable the sustained transdermal or transmucosal absorption of carbon dioxide (Japanese Patent Application Laid-open No. 2000-319187).

However, there still remain some problems in these technologies. For example, the amount of molecular state carbon dioxide dissolved in the water, which is available for transdermal absorption, is limited since the carbon dioxide is in the form of bubbles; it is difficult to keep high concentration of carbon dioxide in the external
preparation since the generation and transdermal absorption of carbon dioxide is not sufficiently controlled; and the carbon dioxide easily leaks into the air.

In order to solve the problems, some compositions were proposed; a composition for preparing an external carbon dioxide preparation comprising a granular material containing a water-soluble acid, a thickener and a water-soluble dispersant as essential components wherein the thickener is mixed with the water-soluble acid and the water-soluble dispersant, and a viscous composition containing a carbonate, water and a thickener as essential components which is to be mixed with the granular material before use (WO 2002/080941 and Japanese Patent Application Laid-open JP-A-2004-307513); a composition of a carbon dioxide external preparation characterized in that carbon dioxide is dissolved in the non-bubble state in the viscous material comprising water and a thickener at least; and a composition for preparing a carbon dioxide external preparation comprising at least a fermentation microorganism, a metabolite of the microorganism, a thickener, water and carbon dioxide (WO 2003/057228). Further proposed is the use of gluconolactone instead of an acid in combination with carbonate for providing a long, durable generation of carbonic acid gas (JP 62 077312 A).

As a result, a composition for preparing a carbon dioxide external preparation comprising non-bubbled carbon dioxide with a type of preparation just before use and a preparation of carbon dioxide external preparation prepared from it are becoming mainstream in these days due to good efficiency of transdermal or transmucosal absorption of carbon dioxide.

Beside, a method for transdermal or transmucosal absorption of carbon dioxide characterized in that the processing to enhance transdermal or transmucosal absorption of carbon dioxide is done before or during the administration of carbon dioxide (WO 2004/078185); materials for formation of carbon dioxide external preparation comprising a base agent wherein the polymeric three-dimensional network structure is impregnated with the viscous material comprising at least an acid and water, being brought into contact with the skin in use, and a reactant comprising a carbonate at least, which generates carbon dioxide upon contact with the base agent above when used and the carbon dioxide dissolves in the viscous material substantially in a non-bubble state (WO 2004/004745); and a carbon dioxide external administration device characterized by comprising a sealing enclosure member capable of sealing a body surface from the ambient air, supply means for supplying carbon dioxide into the inside of the sealing enclosure member and an absorption aid capable of assisting the transdermal or transmucosal absorption of the carbon dioxide inside the sealing enclosure member (WO 2004/002393) are proposed, as well as cosmetic packs wherein gelation occurs between sodium or potassium alginate and calcium chloride which are excellent in removability and can be peeled of (JP 2002 128636 A).

### DISCLOSURE OF THE INVENTION

### PROBLEMS TO BE SOLVED

As described above, carbon dioxide is generated by reacting a water-soluble acid with a carbonate in a prepare-before-use type composition for preparing a carbon dioxide external preparation. However, carbon dioxide is drastically generated if they simply react in the form of powder and the generated carbon dioxide easily leaks into the air, and the efficiency of transdermal or transmucosal carbon dioxide absorption is therefore low.

In order to solve the problem, the water-soluble acid is provided in a granular material (granule) and the carbonate is included in the viscous material in a case of the composition for preparing a carbon dioxide external preparation disclosed in WO 2002/080941. As a result, the rate of disintegration of the granular material and subsequent dissolution of the water-soluble acid in the viscous composition when they are mixed is suitably delayed. Moreover, the generation rate of carbon dioxide has been controlled in a sustained manner using pharmaceutical techniques. For example, the reaction rate between the water-soluble acid and the carbonate is suitably delayed by a thickener(s) in the viscous material.

However, the easy bubble-formation of carbon dioxide still remains a problem although the pharmaceutical techniques can prevent the carbon dioxide from leaking into the air to some extent. Additionally, the pharmaceutical techniques need the water-soluble acid, a dispersant, a binder and a solvent being formulated in granules and further the granules must be dried so as to contain the solvent in a desired percent.

Accordingly, it was a problem that the reaction rate between the water-soluble acid and the carbonate was difficult to be adjusted in a desired range and a lot of time and expenses were required to produce the product. Moreover, it was also a problem that generation of carbon dioxide in a uniform concentration in the viscous composition was difficult when granules were used.

Furthermore, there still remain the following problems; the carbon dioxide external preparation prepared from the composition for preparing a carbon dioxide external preparation is an adhesive viscous fluid and difficult to be removed from the skin or mucosa; and the composition is often dissolved out with blood and/or exudates, which spoils the clothes and the like as well as makes the wound-protection insufficient when it is applied onto the wound surface or the like.

The present invention was achieved in order to solve the problems above, and it is an objective thereof to provide a composition for preparing a carbon dioxide external preparation, which enables it easy to prepare a carbon dioxide external preparation having enhanced aesthetic and medical effects since a sufficient amount of carbon dioxide is contained and the carbon dioxide is transdermally or transmucosally absorbed continuously, and further the production cost of which is reduced.

### MEANS TO SOLVE THE PROBLEM

The present disclosure comprises a composition for preparing a carbon dioxide external preparation characterized by comprising a substance generating an acid after being hydrolyzed, a carbonate, a thickener, and water as essential components.

The present disclosure also comprises a composition for preparing a carbon dioxide external preparation above wherein components except water (a substance generating an acid after being hydrolyzed, a carbonate, and a thickener) form a granular material.

The present disclosure also comprises a composition for preparing a carbon dioxide external preparation above further comprising a dispersant.

Moreover, the present disclosure comprises a composition for preparing a carbon dioxide external preparation above wherein a substance generating an acid after being hydrolyzed and/or a carbonate form a granular material and at least a thickener and water form a viscous material.

The present disclosure comprises a composition for preparing a carbon dioxide external preparation above wherein components except water are included in a water-absorbing support material in a condition not to contact with water before use as a carbon dioxide external preparation.

The present invention is a composition for preparing a carbon dioxide external preparation characterized by comprising a substance generating an acid after being hydrolyzed, a carbonate, a thickener, water, a gelating agent being gelated by calcium ion, and a water-insoluble or poorly soluble calcium salt as defined in the claims.

The present invention is a composition for preparing a carbon dioxide external preparation above wherein components except water (a substance generating an acid after being hydrolyzed, a carbonate, a thickener, a gelating agent being gelated by calcium ion, and a water-insoluble or poorly soluble calcium salt) form a granular material.

The present invention is a composition for preparing a carbon dioxide external preparation above further comprising a dispersant.

The present invention is a composition for preparing a carbon dioxide external preparation above wherein one or more component(s) selected from a substance generating an acid after being hydrolyzed, a carbonate, a gelating agent being gelated by calcium ion, and a water-insoluble or poorly soluble calcium salt form(s) a granular material, and at least a thickener and water form a viscous material.

The present invention is a composition for preparing a carbon dioxide external preparation above wherein the gelating agent being gelated by calcium ion is one or more agent(s) selected from sodium alginate, carrageenan, tara gum, and locust bean gum.

The present invention is a composition for preparing a carbon dioxide external preparation above wherein the water-insoluble or poorly soluble calcium salt above is one or more salt(s) selected from calcium carbonate, calcium sulfate, calcium citrate, calcium alginate, calcium gluconate, calcium pyrophosphate, monobasic calcium phosphate and calcium silicate.

The present invention is a composition for preparing a carbon dioxide external preparation wherein the thickener and the gelating agent being gelated by calcium ion are sodium alginate.

The present invention is a composition for preparing a carbon dioxide external preparation above wherein the carbonate salt and the water-insoluble or poorly soluble calcium salt above are calcium carbonate.

The present invention is a composition for preparing a carbon dioxide external preparation above which provides a carbon dioxide external preparation in the state of coagulated hydrogel.

The present invention is a composition for preparing a carbon dioxide external preparation above wherein the substance generating an acid after being hydrolyzed is one or more selected from lactone, cyclic dimer of organic acid, and acid anhydride.

The present disclosure comprises a composition for preparing a carbon dioxide external
preparation above wherein the substance generating an acid after being hydrolyzed is one or more selected from lactone, cyclic dimer of organic acid, and acid anhydride, in the state of crystal.

The present invention is a composition for preparing a carbon dioxide external preparation above wherein the lactone is glucono-delta-lactone and/or pantolactone.

The present invention is a composition for preparing a carbon dioxide external preparation above wherein the cyclic dimer of organic acid is D,L- or L-lactide (3,6-dimethyl-1,4-dioxane-2,5-dione) and/or D,L- or L-glycolide.

The present invention is a composition for preparing a carbon dioxide external preparation above wherein the acid anhydride above is one or more selected from phthalic anhydride, maleic anhydride, and succinic anhydride.

The present invention is a material for treating a wound or burn comprising a carbon dioxide external preparation obtained from the composition for preparing a carbon dioxide external preparation above.

The present disclosure comprises a pack cosmetic comprising a carbon dioxide external
preparation obtained from the composition for preparing a carbon dioxide external preparation above.

In the composition for preparing a carbon dioxide external preparation of the present invention utilizing a chemical reaction, generating rate of carbon dioxide is controlled by a simple method and it is possible to prepare a carbon dioxide external preparation with enhanced aestheic and medical effects containing a sufficient amount of carbon dioxide, which is transdermally or transmucosally absorbed in a sustained manner. Moreover, a composition for preparing a carbon dioxide external preparation with reduced production cost can be obtained.

Further, a carbon dioxide external preparation which is coagulable within a specified time after being applied, can be obtained if a composition for preparing a carbon dioxide external preparation comprising a gelating agent gelated by calcium ion and a water-insoluble or poorly soluble calcium salt is used. Therefore, the carbon dioxide external preparation is not only easily removed from the skin or mucosa after used, but never dissolved out with blood and/or exudates, which spoils the clothes after or during use when applied onto the wound surface or the like. Furthermore, the coagulated carbon dioxide external preparation may protect the wound surface or the like as a wound dressing after generation of carbon dioxide is terminated.

Subject-matter which is not encompassed by the scope of the claims does not form part of the present claimed invention.

### BEST MODE FOR CARRYING OUT THE INVENTION

Disclosed is a composition for preparing a carbon dioxide external preparation comprising a substance generating an acid after being hydrolyzed, a carbonate, a thickener, and water as essential components (hereinafter, composition for preparing a carbon dioxide external preparation [1]).

The present invention provides a composition for preparing a carbon dioxide external preparation characterized by comprising a gelating agent gelated by calcium ion, and a water-insoluble or poorly soluble calcium salt (hereinafter, composition for preparing a carbon dioxide external preparation [2]) as well as the essential components above (a substance generating an acid after being hydrolyzed, a carbonate, a thickener, and water), which provides a coagulated hydrogel.

The present invention is explained below being classified into the composition for preparing a carbon dioxide external preparation not according to the invention [1] and the composition for preparing a carbon dioxide external preparation according to the invention [2] described above.

The composition for preparing a carbon dioxide external preparation [1] of the present disclosure is characterized by comprising a substance generating an acid after being hydrolyzed, a carbonate, a thickener, and water as essential components.

The substance generating an acid after being hydrolyzed in the present disclosure is preferably one or more selected from lactone, a cyclic dimer of organic acid, and acid anhydride, and more preferably one or more selected from the crystalline forms of lactone, a cyclic dimer of organic acid, and acid anhydride.

Substances generating an acid after being hydrolyzed according to the invention are selected from glucono-delta-lactone; pantolactone; D,L- or L-lactide (3,6-dimethyl-1,4-dioxane-2,5-dione); D,L- or L-glycolide; phthalic anhydride; maleic anhydride; and succinic anhydride. One of these examples or a combination of two or more of these examples may be used. Maleic anhydride and succinic anhydride are preferably used in combination with other substances generating an acid after being hydrolyzed such as glucono-delta-lactone or D,L-lactide to control the generating rate of an acid and consequently to control the generating rate of carbon dioxide at user's option since maleic anhydride and succinic anhydride are easily hydrolyzed and quickly generate an acid.

Any carbonate which generates carbon dioxide by the reaction with an acid may be used as a carbonate in the present invention, and examples of the carbonate include ammonium carbonate, ammonium bicarbonate, potassium carbonate, potassium bicarbonate, potassium sesquicarbonate, sodium carbonate, sodium bicarbonate, sodium sesquicarbonate, lithium carbonate, lithium bicarbonate, lithium sesquicarbonate, cesium carbonate, cesium bicarbonate, cesium sesquicarbonate, magnesium carbonate, magnesium bicarbonate, calcium bicarbonate, calcium carbonate, magnesium hydroxide carbonate, or barium carbonate. One or more of these carbonates can be used and a water-soluble carbonate such as sodium bicarbonate or sodium carbonate is preferable.

There are no particular limitations on thickeners used in the present invention and one or more selected from natural polymers, semi-synthetic polymers, synthetic polymers, and inorganic materials can be used.

Among them, examples of neutral or alkaline thickeners include the followings.

Examples of the natural polymers above include plant-originated polymers such as gum arabic, carrageenan, galactan, agar, quince seed, guar gum, tragacanth gum, mannan, locust bean gum, wheat starch, rice starch, tara gum, corn starch, and potato starch; microorganism-originated polymers such as curdlan, xanthan gum, succinoglucan, dextran, and pullulan; and protein polymers such as albumin, casein, collagen, gelatin and fibroin; one or more of these polymers can be used.

Examples of the semi-synthetic polymers above include
cellulose polymers such as ethylcellulose, carboxymethylcellulose and its salts, carboxymethylethylcellulose and its salts, carboxymethyl starch and its salts, croscarmellose and its salts, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose, and methylhydroxypropylcellulose; starch polymers such as pregelatinaized starch, partially pregelatinaized starch, carboxymethyl starch, dextrin, and methyl starch; alginate polymers such as sodium alginate, potassium alginate, ammonium alginate and propylene glycol alginate; and other polysaccharide polymers such as sodium chondroitin sulfate and sodium hyaluronate; one or more of these polymers can be used.

Examples of the synthetic polymers above include
sodium polyacrylate, polyvinylacetaldiethylaminoacetate, polyvinyl alcohol, polyvinyl pyrrolidone, methacrylic acid-ethyl acrylate copolymer, methacrylic acid - ethyl methacrylate copolymer, ethyl methacrylate - trimethylammoniumethyl chloride methacrylate copolymer, dimethylaminoethyl methacrylate-methyl methacrylate copolymer and the like. One or more of these polymers can be used.

Examples of the inorganic materials above include
hydrated silicon dioxide, colloidal alumina, bentonite, laponite and the like; one or more of these polymers can be used.

Examples of acidic thickeners include the followings.

Alginic acid, pectin and hyaluronic acid are included as a natural polymer; carboxyvinyl polymer is included as a semi-synthetic polymer and light anhydrous silicic acid is included as an inorganic material; one or more of these polymers or a material can be used.

There are no particular limitations on water used in the present invention and natural water, tap water, distilled water, purified water and the like can be used.

A source of carbon dioxide in the composition for preparing a carbon dioxide external preparation of the present invention is the substance generating an acid after being hydrolyzed and the carbonate. The substance generating an acid after being hydrolyzed is gradually hydrolyzed to generate an acid when contacted with water in the present invention, and the acid reacts with the carbonate to generate carbon dioxide gradually. Next, the viscous material is formed by the thickener and the water included in the composition for preparing a carbon dioxide external preparation as essential components. Since the rate of hydrolysis of the substance generating an acid after being hydrolyzed and of generating carbon dioxide subsequent to the hydrolysis is slower in the viscous material than in water, carbon dioxide is generated gradually and constantly, and it provides the carbon dioxide external preparation in which the generated carbon dioxide is difficult to leak into the air.

In the composition for preparing a carbon dioxide external preparation of type [1], the composition for preparing a carbon dioxide external preparation is preferable, wherein a granular material is formed by components other than water (a substance generating an acid after being hydrolyzed, a carbonate and a thickener) (hereinafter, the composition for preparing a carbon dioxide external preparation [1-1]), or one or more component(s) selected from a substance generating an acid after being hydrolyzed, a carbonate, a gelating agent gelated by calcium ion and a water-insoluble or poorly soluble calcium salt form(s) a granulated material, and further at least a thickener and water form a viscous material (hereinafter, the composition for preparing a carbon dioxide external preparation [1-2]). In addition, a granular material in this invention means solid material such as powder, fine granule, granule, crystal and the like or a mixture of these solid materials.

The composition for preparing a carbon dioxide external preparation [1-1] above is comprised of a granular material, which was prepared in advance using an usual technique, comprising a substance generating an acid after being hydrolyzed, a carbonate and a thickener (hereinafter, the granular material [A]) and water. The granular material [A] is mixed with water to provide the carbon dioxide external preparation at use.

The granular material [A] and water are kept without contact before use and the carbon dioxide external preparation is easily obtained when the granular material [A] and water are mixed at use. The granular material [A] can be easily prepared if a substance generating an acid, a carbonate and a thickener are simply mixed for example. It is preferable that the substance generating an acid, the carbonate and the thickener are mixed as uniformly as possible.

A dispersant is preferably included in the granular material [A] so that the thickener is dissolved or uniformly dispersed in water when it is mixed with water by forming less so-called "DAMA" or "MAMAKO" . The term "DAMA" or "MAMAKO" means an aggregate including no water inside, which is formed by aggregation of the solid thickener, and whose surface is covered with a dissolved or swelled viscous substance when the thickener is mixed with water.

There are no particular limitations on the dispersant above, so long as it is easily soluble in water, chemically stable and can be used in a form of granule, and examples thereof include a starch derivative such as pregelatinized starch, α-cyclodextrin and the like; a sugar derivative such as sugar, glucose, fructose, sucrose, lactose, xylitol, D-sorbitol, D-mannitol and the like; a polysaccharide such as pullulan, xanthan gum and the like; a cellulose derivative and salt thereof such as hydroxypropylcellulose, hydroxypropylmethylcellulose, carmellose calcium , carmellose sodium and the like; a synthetic polymer such as polyvinyl pyrrolidone; and urea etc; one or more of these material(s) can be used.

Moreover, additives other than the dispersant above which are commonly used in medicines or cosmetics may be added so that the thickener is easily dissolved or uniformly dispersed in water.

In the composition for preparing a carbon dioxide external preparation [1-2], it is preferable that the substance generating an acid after being hydrolyzed and/or the carbonate form(s) a granular material, and further at least the thickener and water form a viscous material, wherein the thickener is dissolved or uniformly dispersed in water.

If the thickener and water is not sufficiently mixed, the viscosity of the carbon dioxide external preparation obtained is so deficient that the generated carbon dioxide leaks into the air and the amount of transdermally or transmucosally absorbed carbon dioxide may be decreased or the carbon dioxide external preparation may droop down from the skin or mucosa. Therefore it is preferable that the thickener and water form a viscous material which is to be mixed with a granular material at use to prepare the carbon dioxide external preparation having sufficient viscosity.

Examples of a combination between the granular material and the viscous material are as follows;
(1) a viscous material comprising a carbonate, a thickener and water (hereinafter, the viscous material [B]) and a granular material comprising a substance generating an acid after being hydrolyzed (hereinafter, the granular material [B]); and
(2) a viscous material comprising a thickener and water (hereinafter, the viscous material [C]) and a granular material comprising a substance generating an acid after being hydrolyzed and a carbonate (hereinafter, the granular material [C]).
   In a case of the viscous material [B], wherein a thickener is blended in the viscous material combined with a carbonate, the neutral or alkaline thickener which is used in the composition for preparing a carbon dioxide external preparation [1] described before is preferably used. The reason comes from the concern that a carbonate may react with the thickener to generate a carbon dioxide when preparing the viscous material if the thickener is acidic.
   In a case of the viscous material [C], the same thickener used in the composition for preparing a carbon dioxide external preparation [1] (the neutral or alkaline, or acidic thickener above) can be used.
   In the composition for preparing a carbon dioxide external preparation [1], all the components are preferably mixed when the carbon dioxide external preparation is at use in either case of the composition for preparing a carbon dioxide external preparation [1-1] or the composition for preparing a carbon dioxide external preparation [1-2]
   When preparing the composition for preparing a carbon dioxide external preparation [1-1] using a water-absorbing support material, the granular material [A] is preferably contained in the support material not being contacted with water before use. In such case, the carbon dioxide external preparation can be used as a material for wound remedy or pack cosmetic, if only the water-absorbing support material is soaked in or moistened with water. The water-absorbing support material is also used as a sheet for treating a wound since carbon dioxide is generated by wound exudate if the support material is directly applied to the wound.
   There are no particular limitations on the water-absorbing support material above, so long as it is possible to contain the granular material [A], having water absorbing capacity, and applicable to a skin. The water-absorbing support material may be any of a woven fabric, a nonwoven fabric, a sponge or the like, and can be selected as appropriate in accordance with the specific purpose, site of application and so on. Among them, a nonwoven fabric is light, and has excellent capacity of keeping the granular material and is thus particularly preferable.
   Next, the composition for preparing a carbon dioxide external preparation [2] of the present invention is explained in detail.
   The composition for preparing a carbon dioxide external preparation [2] is a composition characterized by comprising a substance generating an acid after being hydrolyzed, a carbonate, a thickener, water, a gelating agent gelated by calcium ion, and a water-insoluble or poorly soluble calcium salt.
   In a case of the composition for preparing a carbon dioxide external preparation [2], the substance generating an acid after being hydrolyzed is hydrolyzed to generate an acid, which reacts with the carbonate to generate carbon dioxide. Simultaneously, the acid also reacts with the water-insoluble or poorly soluble calcium salt to generate calcium ion, which causes gelation of the gelating agent gelated by calcium ion to provide a solidified hydrogel. As a result, the carbon dioxide external preparation obtained from the composition for preparing a carbon dioxide external preparation [2] has an effect that the preparation is easily removed from the skin or mucosa after used as well as the merits described above.
   When the carbon dioxide external preparation is applied to a wound surface or the like, there is a merit that the preparation is hardly dissolved out with blood and/or exudates and additionally can protect the wound as a wound dressing after generation of carbon dioxide is terminated. If it is used for cosmetic purpose, the preparation has advantages of being removed easily after use and never spoiling the clothes.
   The substance generating an acid after being hydrolyzed, the carbonate, the thickener, and the water, which are used in the composition for preparing a carbon dioxide external preparation [1] described above, are also used in the same manner as essential components of the composition for preparing a carbon dioxide external preparation [2]
   One or more selected from sodium alginate, carrageenan, tara gum and locust bean gum is (are) preferably used as the gelating agent gelated by calcium ion (hereinafter, also referred to as the gelating agent) in the composition for preparing a carbon dioxide external preparation [2] above.
   One or more selected from calcium carbonate, calcium sulfate, calcium citrate, calcium alginate, calcium gluconate, calcium pyrophosphate, monobasic calcium phosphate, dicalcium phosphate and calcium silicate is (are) preferably used as the water-insoluble or poorly soluble calcium salt in the composition for preparing a carbon dioxide external preparation [2] above.
   If a water-soluble calcium salt is used, the rate of reaction between a gelating agent and calcium ion becomes faster than the rate of generating carbon dioxide by the reaction of a substance generating an acid after being hydrolyzed with a carbonate and the carbon dioxide external preparation is coagulated within a short period. As a result, the generation of carbon dioxide is terminated in the middle of the reaction thereof and so the use of the water-soluble calcium salt is not preferred. Examples of the carbonate and the slightly soluble calcium salt include calcium carbonate, which is preferably used because it is a source of carbon dioxide and calcium ion at a time.
   In the composition for preparing a carbon dioxide external preparation [2] of the present invention, the composition for preparing a carbon dioxide external preparation is preferable, wherein a granular material is formed by components other than water (a substance generating an acid after being hydrolyzed, a carbonate, a thickener, a gelating agent gelated by calcium ion and a water-insoluble or poorly soluble calcium salt) (hereinafter, the composition for preparing a carbon dioxide external preparation [2-1]), or one or more components selected from a substance generating an acid after being hydrolyzed, a carbonate, a gelating agent gelated by calcium ion and a water-insoluble or poorly soluble calcium salt form(s) a granular material and further at least a thickener and water form a viscous material (hereinafter, the composition for preparing a carbon dioxide external preparation [2-2]), in the same manner of the composition for preparing a carbon dioxide external preparation [1].
   The composition for preparing a carbon dioxide external preparation [2-1] above is comprised of a granular material, which was prepared in advance using a common technique, comprising a substance generating an acid after being hydrolyzed, a carbonate, a thickener, a gelating agent and a water-insoluble or poorly soluble calcium salt (hereinafter, the granular material [D]) and water. The granular material [D] is mixed with water to provide the carbon dioxide external preparation at use.
   The granular material [D] and water are kept without contact before use and the carbon dioxide external preparation is easily obtained when the granular material [D] and water are mixed at use. The granular material [D] can be easily prepared if a substance generating an acid, a carbonate, a thickener, water, a gelating agent gelated by calcium ion and a water-insoluble or poorly soluble calcium salt are simply mixed for example. It is preferable that the substance generating an acid, the carbonate, the thickener, water, the gelating agent gelated by calcium ion and the water-insoluble or poorly soluble calcium salt are mixed as uniformly as possible.
   Also in the composition for preparing a carbon dioxide external preparation [2-1], it is preferable that a dispersant is further included in the granular material [D] in the same manner as the composition for preparing a carbon dioxide external preparation [1-1]. The same dispersant as that of the composition for preparing a carbon dioxide external preparation [1-1] can be used.
   In the composition for preparing a carbon dioxide external preparation [2-2], it is preferable that a granular material is formed by one or more selected from a substance generating an acid after being hydrolyzed, a carbonate, a gelating agent gelated by calcium ion and a water-insoluble or poorly soluble calcium salt, and further at least a thickener and water form a viscous material, wherein the thickener is in a state of being dissolved or uniformly dispersed in water.
   Examples of the combination between the viscous material above and the granular material above are shown below.
(3) a viscous material comprising a carbonate, a thickener, a gelating agent, a water-insoluble or poorly soluble calcium salt and water (hereinafter, the viscous material [E]) and a granular material comprising a substance generating an acid after being hydrolyzed (hereinafter, the viscous material [E]);
(4) a viscous material comprising a thickener, a gelating agent, a water-insoluble or poorly soluble calcium salt and water (hereinafter, the viscous material [F]) and a granular material comprising a substance generating an acid after being hydrolyzed and a carbonate (hereinafter, the granular material [F]);
(5) a viscous material comprising a thickener, a water-insoluble or poorly soluble calcium salt and water (hereinafter, the viscous material [G]) and a granular material comprising a substance generating an acid after being hydrolyzed, a carbonate and a gelating agent (hereinafter, the granular material [G]);
(6) a viscous material comprising a carbonate, a thickener, a water-insoluble or poorly soluble calcium salt and water (hereinafter, the viscous material [H]) and a granular material comprising a substance generating an acid after being hydrolyzed, and a gelating agent (hereinafter, the granular material [H]);
(7) a viscous material comprising a carbonate, a thickener, a gelating agent and water (hereinafter, the viscous material [I]) and a granular material comprising a substance generating an acid after being hydrolyzed and a water-insoluble or poorly soluble calcium salt (hereinafter, the granular material [I]);
(8) a viscous material comprising a thickener, a gelating agent, and water (hereinafter, the viscous material [J]) and a granular material comprising a substance generating an acid after being hydrolyzed, a carbonate, and a water-insoluble or poorly soluble calcium salt (hereinafter, the granular material [J]);
(9) a viscous material comprising a carbonate, a thickener and water (hereinafter, the viscous material [K]) and a granular material comprising a substance generating an acid after being hydrolyzed, a gelating agent and a water-insoluble or poorly soluble calcium salt (hereinafter, the granular material [K]); and
(10) a viscous material comprising a thickener and water (hereinafter, the viscous material [L]) and a granular material comprising a substance generating an acid after being hydrolyzed, a carbonate, a gelating agent and a water-insoluble or poorly soluble calcium salt (hereinafter, the granular material [L]).

A neutral or alkaline thickener is preferably used as the thickener forming the viscous material [E], [F], [G], [H], [I] and [K]. As the neutral or alkaline thickener, the same thickener as that of the viscous material [B] can be used.

There are no particular limitations on the thickeners forming the viscous material [J] or [L] above, and the same thickener as that of the composition for preparing a carbon dioxide external preparation [1] (the neutral or alkaline, or acidic thickener described above) can be used.

When the composition for preparing a carbon dioxide external preparation [2-1] is prepared using a water-absorbing support material, it is preferable that the granular material [D] is contained in the water-absorbing support material not being contacted with water before use. In such case, the carbon dioxide external preparation can be used as a material for wound remedy or pack cosmetic, if only the water-absorbing support material is soaked in or moistened with water. The water-absorbing support material is also used as a sheet for treating a wound since carbon dioxide is generated by wound exudate if the support material is directly applied to the wound. As the water-absorbing support material, the same material as that of the composition for preparing a carbon dioxide external preparation [1-1] can be used.

A mixing ratio of the essential components included in the composition for preparing a carbon dioxide external preparation [1] is shown below compared to the whole composition; the substance generating an acid after being hydrolyzed is preferably 0.5 to 30 mass %, more preferably 1 to 20 mass %, the carbonate is preferably 0.3 to 10 mass %, more preferably 1 to 5 mass %, the thickener is preferably 0.5 to 10 mass %, more preferably 1 to 7 mass % and the water is preferably 60 to 95 mass %, more preferably 75 to 93 mass %.

According to the invention, a mixing ratio of the essential components included in the composition for preparing a carbon dioxide external preparation [2] is shown below compared to the whole composition; the substance generating an acid after being hydrolyzed is 0.5 to 40 mass %, more preferably 1 to 35 mass %, the carbonate is 0.1 to 10 mass %, more preferably 0.3 to 5 mass %, the thickener is 0.5 to 10 mass %, more preferably 1 to 7 mass %, the water is 40 to 95 mass %, more preferably 50 to 93 mass %, the gelating agent is 0.5 to 10 mass %, more preferably 1 to 7 mass % and the water-insoluble or poorly soluble calcium salt is 0.1 to 5 mass %, more preferably 0.2 to 3 mass %.

The granular material used in the composition for preparing a carbon dioxide external preparation of the present invention is available in a form of a mixed powder by mixing each component using a mixer which is common in the manufacturing of medicines or cosmetics as described above. Fine granule or granule is also available by granulating each component by common granulating techniques (e.g., a compression molding technique, an extruding granulation technique, a tumbling granulation technique, a spray granulation technique, an agitating granulation technique and the like) for manufacturing medicines.

The viscous material used in the composition for preparing a carbon dioxide external preparation of the present invention can be prepared by dissolving or uniformly dispersing a thickener, water and other components using a homogenizer which is common in the manufacturing of external medicines or cosmetics.

In the carbon dioxide external preparation of the present invention, materials commonly used in ordinary external preparations or cosmetics, for example, colorants, spreading agents, preservatives, surfactants, oils, moisturizers, alcohols, antioxidants, sequestering agents, anti-colorants, ultraviolet absorbing/scattering agents, vitamins, amino acids, melanin pigment synthesis inhibiting agents, nutrients, antiinflammatories, vasodilators, hormones, astringents, antihistamines, sebum inhibiting agents, keratin stripping/dissolving agents, anti-seborrheic agents, anti-itching agents and the like can be added if necessary into the granular material or the viscous material above as well as the essential components described above so long as this is within a range such that the effects of the present invention are not impaired, whereby the carbon dioxide external preparation can be used yet more effectively as a cosmetic or an external medicine.

In the composition for preparing a carbon dioxide external preparation not according to the invention [1] and the composition for preparing a carbon dioxide external preparation according to the invention [2], the blending ratio between the substance generating an acid after being hydrolyzed and the carbonate may be selected properly so that the carbon dioxide external preparation prepared by mixing all the components generates carbon dioxide continuously. It depends on the types of the substance generating an acid after being hydrolyzed, the carbonate and the thickener, and the mixing ratio between the thickener and the water, but the ratio between the acid equivalence of the acid generated by the hydrolysis of the substance generating an acid after being hydrolyzed and the base equivalence of the carbonate is preferably in a range of 0.5:1 to 40:1. Further, the weight ratio of the carbon dioxide generated to the whole composition is preferably more than 60 ppm.

The carbon dioxide external preparation obtained from the composition for preparing a carbon dioxide external preparation of the present invention can be used as a material for treating a wound or burn, or as a pack cosmetic. It is preferable to use it for more than five minutes and there is no upper limit. As for frequency of use, it is preferable to use it once a day, or once in several days.

The granular material and the water, or the granular material and the viscous material of the present invention may be mixed in a suitable vessel made of glass or plastic, or on the palm or the skin or mucosa.

As a preservation method of the composition for preparing a carbon dioxide external preparation of the present invention, it is only necessary for the granular material and the water, or the granular material and the viscous material to be kept without contact, and they are preferably preserved in a well-sealed condition. There is no particular limitations on material or form of the preservation vessel, and examples of the material include plastic, glass, aluminum, paper, various polymers, complex of these materials and the like; examples of the form include a cup, a tube, a bag, a bottle, a stick, a pump and the like.

The present invention is concretely illustrated using examples and comparative examples. Examples 1-8, 28 and Test examples 1 and 2 are reference examples. In addition, mass % in the examples means % compared with the whole compositions unless they are particularly specified.

### Example 1 Preparation of the composition for preparing a carbon dioxide external preparation [1-1]

The composition for preparing a carbon dioxide external preparation of Example 1 (5.0 g) was obtained by combining a mixed powder of a substance generating an acid after being hydrolyzed (glucono-delta-lactone, 5.4 mass %), a carbonate (sodium bicarbonate, 1.0 mass %), and a thickener (carrageenan, 5.0 mass %) with water (purified water, 88.6 mass %)

### Example 2 Preparation of the composition for preparing a carbon dioxide external preparation [1-1]

The composition for preparing a carbon dioxide external preparation of Example 2 (5.0 g) was obtained by combining a mixed powder of a substance generating an acid after being hydrolyzed (glucono-delta-lactone, 5.0 mass %), a carbonate (sodium bicarbonate, 1.0 mass %), and a thickener (tragacanth gum, 4.0 mass %) with water (purified water, 90.0 mass %)

### Example 3 Preparation of the composition for preparing a carbon dioxide external preparation [1-1]

The composition for preparing a carbon dioxide external preparation of Example 3 (5.0 g) was obtained by combining a mixed powder of a substance generating an acid after being hydrolyzed (glucono-delta-lactone, 5.0 mass %), a carbonate (sodium bicarbonate, 1.0 mass %), and a thickener (tara gum, 4.0 mass %) with water (purified water, 90.0 mass %)

### Example 4 Preparation of the composition for preparing a carbon dioxide external preparation [1-1]

The composition for preparing a carbon dioxide external preparation of Example 4 (5.0 g) was obtained by combining a mixed powder of a substance generating an acid after being hydrolyzed (glucono-delta-lactone, 5.2 mass %), a carbonate (sodium bicarbonate, 1.0 mass %), and a thickener (carrageenan, 2.0 mass % and locust bean gum, 1.0 mass %) with water (purified water, 90.8 mass %).

### Example 5 Preparation of the composition for preparing a carbon dioxide external preparation [1-1]

The composition for preparing a carbon dioxide external preparation of Example 5 (20.0 g) was obtained by combining a mixed powder of a substance generating an acid after being hydrolyzed (glucono-delta-lactone, 5.2 mass %), a carbonate (sodium bicarbonate, 1 mass %), a thickener (carrageenan, 2 mass % and locust bean gum, 2 mass %) and a dispersant (glucose, 20 mass %) with water (purified water, 69.8 mass %).

### Example 6 Preparation of the composition for preparing a carbon dioxide external preparation [1-1]

The composition for preparing a carbon dioxide external preparation of Example 6 was obtained by combining a water-absorbing support material (cotton nonwoven fabric of 3 cm x 3 cm) containing a granular material comprising a mixed powder of a substance generating an acid after being hydrolyzed (glucono-delta-lactone, 4.3 mass %), a carbonate (sodium bicarbonate, 0.7 mass %) and a thickener (sodium alginate, 2.1 mass %) with water (purified water, 0.65 g, 92.9 mass %).

### Example 7 Preparation of the composition for preparing a carbon dioxide external preparation [1-2]

The composition for preparing a carbon dioxide external preparation of Example 7 was obtained by combining a substance generating an acid after being hydrolyzed (glucono-delta-lactone, 0.199 g, 6.2 mass %) with a viscous material (3.0 g) which was obtained by sufficiently mixing a carbonate (sodium carbonate, 0.9 mass %), a thickener (sodium carboxymethylcellulose, 2.3 mass % and sodium alginate, 1.8 mass %), a spreading agent (1,3-butyleneglycol, 3.8 mass %, and glycerin, 3.8 mass %), a preservative (pentyleneglycol, 2.8 mass % and phenoxyethanol, 0.5 mass %) and water (purified water, 77.9 mass %).

### Example 8 Preparation of the composition for preparing a carbon dioxide external preparation [1-2]

The composition for preparing a carbon dioxide external preparation of Example 8 was obtained by combining a granular material (0.23 g) comprising a mixed powder of a substance generating an acid after being hydrolyzed (glucono-delta-lactone, 6.2 mass %) and a carbonate (sodium bicarbonate, 0.9 mass %) with a viscous material (3.0 g) which was obtained by sufficiently mixing a thickener (sodium carboxymethylcellulose, 0.8 mass % and sodium alginate, 3.3 mass %), a spreading agent (1,3-butyleneglycol, 6.5 mass %), a preservative (pentyleneglycol, 2.5 mass % and phenoxyethanol, 0.5 mass %) and water (purified water, 89.3 mass %).

### Example 9 Preparation of the composition for preparing a carbon dioxide external preparation [2-1]

The composition for preparing a carbon dioxide external preparation of Example 9 (1.6 g) was obtained by combining a mixed powder of a substance generating an acid after being hydrolyzed (glucono-delta-lactone, 2.1 mass %), a carbonate (sodium bicarbonate, 0.5 mass %), and a thickener (also workable as a gelating agent) (sodium alginate, 2.5 mass %) and a water-insoluble or poorly soluble calcium salt (calcium citrate, 1.1 mass %) with water (purified water, 93.8 mass %).

### Example 10 Preparation of the composition for preparing a carbon dioxide external preparation [2-1]

The composition for preparing a carbon dioxide external preparation of Example 10 (2.6 g) was obtained by combining a mixed powder of a substance generating an acid after being hydrolyzed (glucono-delta-lactone, 2.3 mass %), a carbonate (sodium bicarbonate, 0.6 mass %), and a thickener (also workable as a gelating agent) (sodium alginate, 2.8 mass %), a water-insoluble or poorly soluble calcium salt (calcium silicate, 0.8 mass %) and a dispersant (lactose, 16.6 mass %) with water (purified water, 76.9 mass %).

### Example 11 Preparation of the composition for preparing a carbon dioxide external preparation [2-1]

The composition for preparing a carbon dioxide external preparation of Example 11 was obtained by combining a water-absorbing support material (cotton nonwoven fabric of 3 cm x 3 cm) containing a granular material comprising a mixed powder of a substance generating an acid after being hydrolyzed (glucono-delta-lactone, 2.03 mass %), a carbonate (sodium bicarbonate, 0.19 mass % and calcium carbonate (also workable as a water-insoluble or poorly soluble calcium salt), 0.27 mass %), a gelating agent (also workable as a thickener) (sodium alginate, 1.22 mass %), a thickener (sodium carboxymethylcellulose, 0.48 mass %), a preservative (methyl paraben, 0.05 mass %) and a dispersant (glucose, 4.85 mass %) with water (purified water, 0.5 g, 90.91 mass %).

### Example 12 Preparation of the composition for preparing a carbon dioxide external preparation [2-1]

The composition for preparing a carbon dioxide external preparation of Example 12 was obtained by combining a water-absorbing support material (cotton nonwoven fabric of 3 cm x 3 cm) containing a granular material (0.05 g) comprising a mixed powder of a substance generating an acid after being hydrolyzed (glucono-delta-lactone, 2.03 mass %), a carbonate (sodium bicarbonate, 0.19 mass % and calcium carbonate (also workable as a water-insoluble or poorly soluble calcium salt), 0.27 mass %), a gelating agent (also workable as a thickener) (sodium alginate, 1.35 mass %), a thickener (sodium carboxymethylcellulose, 0.35 mass %), a preservative (methyl paraben, 0.05 mass %) and a dispersant (glucose, 4.85 mass %) with water (purified water, 0.5 g, 90.91 mass %).

### Example 13 Preparation of the composition for preparing a carbon dioxide external preparation [2-2]

The composition for preparing a carbon dioxide external preparation of Example 13 was obtained by combining a substance generating an acid after being hydrolyzed (glucono-delta-lactone, 1.6 g, 34.8 mass %) with a viscous material (3.0 g) which was obtained by sufficiently mixing a carbonate (sodium bicarbonate, 0.7 mass %), a thickener (sodium carboxymethylcellulose, 0.6 mass %), a gelating agent (also workable as a thickener) (tara gum, 2.6 mass %), a water-insoluble or poorly soluble calcium salt (calcium sulfate dihydrate, 2.0 mass %), a spreading agent (1,3-butyleneglycol, 4.6 mass %), a preservative (pentyleneglycol, 1.8 mass % and phenoxyethanol, 0.3 mass %) and water (purified water, 52.6 mass %).

### Example 14 Preparation of the composition for preparing a carbon dioxide external preparation [2-2]

The composition for preparing a carbon dioxide external preparation of Example 14 was obtained by combining a substance generating an acid after being hydrolyzed (glucono-delta-lactone, 1.6 g, 34.8 mass %) with a viscous material (3.0 g) which was obtained by sufficiently mixing a carbonate (sodium bicarbonate, 0.7 mass %), a thickener (sodium carboxymethylcellulose, 0.6 mass %), a gelating agent (also workable as a thickener) (locust bean gum, 2.3 mass %), a water-insoluble or poorly soluble calcium salt (calcium sulfate dihydrate, 2.0 mass %), a spreading agent (1,3-butyleneglycol, 4.6 mass %), a preservative (pentyleneglycol, 1.8 mass % and phenoxyethanol, 0.3 mass %) and water (purified water, 52.9 mass %).

### Example 15 Preparation of the composition for preparing a carbon dioxide external preparation [2-2]

The composition for preparing a carbon dioxide external preparation of Example 15 was obtained by combining a substance generating an acid after being hydrolyzed (glucono-delta-lactone, 1.0 g, 25.0 mass %) with a viscous material (3.0 g) which was obtained by sufficiently mixing a carbonate (calcium carbonate (also workable as a water-insoluble or poorly soluble calcium salt), 2.3 mass %), a thickener (sodium carboxymethylcellulose, 0.7 mass %), a gelating agent (also workable as a thickener) (carrageenan, 3.0 mass %), a spreading agent (1,3-butyleneglycol, 5.3 mass %), a preservative (pentyleneglycol, 2.0 mass % and phenoxyethanol, 0.4 mass %) and water (purified water, 61.3 mass %).

### Example 16 Preparation of the composition for preparing a carbon dioxide external preparation [2-2]

The composition for preparing a carbon dioxide external preparation of Example 16 was obtained by combining a substance generating an acid after being hydrolyzed (glucono-delta-lactone, 0.4 g, 11.8 mass %) with a viscous material (3.0 g) which was obtained by sufficiently mixing a carbonate (calcium carbonate (also workable as a water-insoluble or poorly soluble calcium salt), 0.4 mass % and sodium bicarbonate, 0.9 mass %), a thickener (sodium carboxymethylcellulose, 0.9 mass %), a gelating agent (also workable as a thickener) (sodium alginate, 3.4 mass %), a spreading agent (1,3-butyleneglycol, 5.3 mass %), a preservative (pentyleneglycol, 2.4 mass % and phenoxyethanol, 0.4 mass %) and water (purified water, 74.5 mass %).

### Example 17 Preparation of the composition for preparing a carbon dioxide external preparation [2-2]

The composition for preparing a carbon dioxide external preparation of Example 17 was obtained by combining a granular material (0.63 g) comprising a mixed powder of a substance generating an acid after being hydrolyzed (glucono-delta-lactone, 16.5 mass %) and a carbonate (sodium bicarbonate, 0.8 mass %) with a viscous material (3.0 g) which was obtained by sufficiently mixing a thickener (sodium carboxymethylcellulose, 0.7 mass %), a gelating agent (also workable as a thickener) (sodium alginate, 2.9 mass %), a water-insoluble or poorly soluble calcium salt (calcium citrate tetrahydrate, 0.8 mass %), a spreading agent (1,3-butyleneglycol, 5.8 mass %), a preservative (pentyleneglycol, 2.2 mass % and phenoxyethanol, 0.4 mass %) and water (purified water, 69.9 mass %).

### Example 18 Preparation of the composition for preparing a carbon dioxide external preparation [2-2]

The composition for preparing a carbon dioxide external preparation of Example 18 was obtained by combining a granular material (0.37 g) comprising a mixed powder of a substance generating an acid after being hydrolyzed (glucono-delta-lactone, 4.4 mass %), a carbonate (sodium bicarbonate, 0.6 mass %), and a gelating agent (also workable as a thickener) (sodium alginate, 5.9 mass %) with a viscous material (3.0 g) which was obtained by sufficiently mixing a thickener (sodium carboxymethylcellulose, 0.4 mass %), a water-insoluble or poorly soluble calcium salt (calcium alginate, 1.8 mass %) and water (purified water, 86.9 mass %).

### Example 19 Preparation of the composition for preparing a carbon dioxide external preparation [2-2]

The composition for preparing a carbon dioxide external preparation of Example 19 was obtained by combining a granular material (0.3 g) comprising a mixed powder of a substance generating an acid after being hydrolyzed (glucono-delta-lactone, 4.5 mass %) and a gelating agent (also workable as a thickener) (sodium alginate, 4.5 mass %) with a viscous material (3.0 g) which was obtained by sufficiently mixing carbonate (sodium bicarbonate, 0.6 mass %), a thickener (sodium carboxymethylcellulose, 0.9 mass %), a water-insoluble or poorly soluble calcium salt (calcium silicate, 1.4 mass %) and water (purified water, 88.1 mass %).

### Example 20 Preparation of the composition for preparing a carbon dioxide external preparation [2-2]

The composition for preparing a carbon dioxide external preparation of Example 20 was obtained by combining a granular material (0.86 g) comprising a mixed powder of a substance generating an acid after being hydrolyzed (glucono-delta-lactone, 20.7 mass %) and a water-insoluble or poorly soluble calcium salt (calcium pyrophosphate, 1.6 mass %) with a viscous material (3.0 g) which was obtained by sufficiently mixing a carbonate (sodium bicarbonate, 0.8 mass %), a thickener (sodium carboxymethylcellulose, 0.7 mass %), a gelating agent (also workable as a thickener) (sodium alginate, 2.7 mass %), a spreading agent (1,3-butyleneglycol, 5.4 mass %), a preservative (pentyleneglycol, 2.1 mass % and phenoxyethanol, 0.4 mass %) and water (purified water, 65.6 mass %).

### Example 21 Preparation of the composition for preparing a carbon dioxide external preparation [2-2]

The composition for preparing a carbon dioxide external preparation of Example 21 was obtained by combining a granular material (0.83 g) comprising a mixed powder of a substance generating an acid after being hydrolyzed (glucono-delta-lactone, 20.9 mass %) and a water-insoluble or poorly soluble calcium salt (dibasic calcium phosphate dihydrate, 0.8 mass %) with a viscous material (3.0 g) which was obtained by sufficiently mixing a carbonate (sodium bicarbonate, 0.8 mass %), a thickener (sodium carboxymethylcellulose, 0.7 mass %), a glating agent (also workable as a thickener) (sodium alginate, 2.7 mass %), a spreading agent (1,3-butyleneglycol, 5.5 mass %), a preservative (pentyleneglycol, 2.1 mass % and phenoxyethanol, 0.4 mass %) and water (purified water, 66.1 mass %).

### Example 22 Preparation of the composition for preparing a carbon dioxide external preparation [2-2]

The composition for preparing a carbon dioxide external preparation of Example 22 was obtained by combining a granular material (0.43 g) comprising a mixed powder of a substance generating an acid after being hydrolyzed (glucono-delta-lactone, 11.7 mass %) and a water-insoluble or poorly soluble calcium salt (monobasic calcium phosphate monohydrate, 0.9 mass %) with a viscous material (3.0 g) which was obtained by sufficiently mixing a carbonate (sodium carbonate, 0.9 mass %), a thickener (sodium carboxymethylcellulose, 0.8 mass %), a gelating agent (also workable as a thickener) (sodium alginate, 3.1 mass %), a spreading agent (1,3-butyleneglycol, 6.1 mass %), a preservative (pentyleneglycol, 2.4 mass % and phenoxyethanol, 0.4 mass %) and water (purified water, 73.7 mass %).

### Example 23 Preparation of the composition for preparing a carbon dioxide external preparation [2-2]

The composition for preparing a carbon dioxide external preparation of Example 23 was obtained by combining a granular material (0.49 g) comprising a mixed powder of a substance generating an acid after being hydrolyzed (glucono-delta-lactone, 11.5 mass %), a carbonate (sodium carbonate, 1.7 mass %) and a water-insoluble or poorly soluble calcium salt (calcium gluconate monohydrate, 0.8 mass %) with a viscous material (3.0 g) which was obtained by sufficiently mixing a thickener (sodium carboxymethylcellulose, 0.8 mass %), a gelating agent (also workable as a thickener) (sodium alginate, 3.0 mass %), a spreading agent (1,3-butyleneglycol, 6.0 mass %), a preservative (pentyleneglycol, 2.3 mass % and phenoxyethanol, 0.4 mass %) and water (purified water, 73.5 mass %).

### Example 24 Preparation of the composition for preparing a carbon dioxide external preparation [2-2]

The composition for preparing a carbon dioxide external preparation of Example 24 was obtained by combining a granular material (0.34 g) comprising a mixed powder of a substance generating an acid after being hydrolyzed (glucono-delta-lactone, 4.5 mass %), a gelling agent (also workable as a thickener) (sodium alginate, 4.5 mass %), and a water-insoluble or poorly soluble calcium salt (calcium siliconate, 1.2 mass %) with a viscous material (3.0 g) which was obtained by sufficiently mixing a carbonate (sodium bicarbonate, 0.6 mass %) and a thickener (sodium carboxymethylcellulose, 0.9 mass %) and water (purified water, 88.3 mass %).

### Example 25 Preparation of the composition for preparing a carbon dioxide external preparation [2-2]

The composition for preparing a carbon dioxide external preparation of Example 25 was obtained by combining a granular material (0.36 g) comprising a mixed powder of a substance generating an acid after being hydrolyzed (glucono-delta-lactone, 4.5 mass %), a carbonate (sodium bicarbonate, 0.5 mass %), a gelating agent (also workable as a thickener) (sodium alginate, 4.5 mass %) and a water-insoluble or poorly soluble calcium salt (calcium alginate, 1.2 mass %) with a viscous material (3.0 g) which was obtained by sufficiently mixing a thickener (sodium carboxymethylcellulose, 0.8 mass %), a spreading agent (1,3-butyleneglycol, 6.3 mass %), a preservative (pentyleneglycol, 2.4 mass % and phenoxyethanol, 0.4 mass %) and water (purified water, 79.4 mass %).

### Example 26 Preparation of the composition for preparing a carbon dioxide external preparation [2-2]

The composition for preparing a carbon dioxide external preparation of Example 26 was obtained by combining 1.6 g of a substance generating an acid after being hydrolyzed (glucono-delta-lactone, 6.0 mass %) with a viscous material (25.0 g) which was obtained by sufficiently mixing a gelating agent (also workable as a thickener) (sodium alginate, 3.3 mass %), a water-insoluble or poorly soluble calcium salt (also workable as a carbonate)(calcium carbonate, 0.7 mass %), a carbonate (sodium bicarbonate , 0.5 mass %), a thickener (sodium carboxymethylcellulose, 0.8 mass %), a spreading agent (1,3-butyleneglycol, 6.6 mass %), a preservative (pentyleneglycol, 2.5 mass % and phenoxyethanol, 0.5 mass %) and water (purified water, 79.1 mass %).

### Example 27 Preparation of the composition for preparing a carbon dioxide external preparation [2-2]

The composition for preparing a carbon dioxide external preparation of Example 27 was obtained by combining 0.80 g of a substance generating an acid after being hydrolyzed (glucono-delta-lactone, 6.0 mass %) with a viscous material (12.50 g) which was obtained by sufficiently mixing a gelating agent (also workable as a thickener) (sodium alginate, 1.9 mass %), a water-insoluble or poorly soluble calcium salt (also workable as a carbonate)(calcium carbonate, 47.0 mass % and water (purified water, 45.1 mass %).

### Example 28 Preparation of the composition for preparing a carbon dioxide external preparation [1-2]

The composition for preparing a carbon dioxide external preparation of Example 28 was obtained by combining a substance generating an acid after being hydrolyzed (glucono-delta-lactone, 0.07 g, 4.5 mass %) with a viscous material which was obtained by sufficiently mixing 85.8 mass % of purified water, 1.0 mass % of sodium bicarbonate, 2.9 mass % of sodium alginate, 2.9 mass % of sodium carboxymethylcellulose and 2.9 mass % of 1,2-pentanediol.

### Example 29 Preparation of the composition for preparing a carbon dioxide external preparation [2-1]

The composition for preparing a carbon dioxide external preparation of Example 29 is obtained by combining a mixed powder of a substance generating an acid after being hydrolyzed (D,L-lactide, 2.1 mass %), a carbonate (sodium bicarbonate, 0.5 mass %), a thickener (also workable as a gelating agent)(sodium alginate, 2.5 mass %) and a water-insoluble or poorly soluble calcium salt (calcium citrate, 1.1 mass %) with water (purified water, 93.8 mass %).

### Example 30 Preparation of the composition for preparing a carbon dioxide external preparation [2-1]

The composition for preparing a carbon dioxide external preparation of Example 30 is obtained in the same manner as Example 29 except that the substance generating an acid after being hydrolyzed is replaced with L-glycolide.

### Example 31 Preparation of the composition for preparing a carbon dioxide external preparation [2-1]

The composition for preparing a carbon dioxide external preparation of Example 31 is obtained by combining a mixed powder of a substance generating an acid after being hydrolyzed (pantolactone, 2.3 mass %), a carbonate (sodium bicarbonate, 0.6 mass %), a thickener (also workable as a gelating agent)(sodium alginate, 2.8 mass %), a water-insoluble or poorly soluble calcium salt (calcium silicate, 0.8 mass %) and a dispersant (lactose, 16.6 mass %) with water (purified water, 76.9 mass %).

### Example 32 Preparation of the composition for preparing a carbon dioxide external preparation [2-1]

The composition for preparing a carbon dioxide external preparation of Example 32 is obtained by combining a mixed powder of a substance generating an acid after being hydrolyzed (phthalic anhydride, 2.3 mass %), a carbonate (sodium bicarbonate, 0.6 mass %), a thickener (also workable as a gelating agent)(sodium alginate, 2.8 mass %), a water-insoluble or poorly soluble calcium salt (calcium silicate, 0.8 mass %) and a dispersant (lactose, 16.6 mass %) with water (purified water, 76.9 mass %).

### Example 33 Preparation of the composition for preparing a carbon dioxide external preparation [2-1]

The composition for preparing a carbon dioxide external preparation of Example 33 is obtained in the same manner as Example 32 except that the substance generating an acid after being hydrolyzed is replaced with maleic anhydride. Example 34 Preparation of the composition for preparing a carbon dioxide external preparation [2-1]

The composition for preparing a carbon dioxide external preparation of Example 34 is obtained in the same manner as Example 32 except that the substance generating an acid after being hydrolyzed is replaced with succinic anhydride.

### Example 35 Preparation of the composition for preparing a carbon dioxide external preparation [2-2]

The composition for preparing a carbon dioxide external preparation of Example 35 was obtained by combining a substance generating an acid after being hydrolyzed (glucono-delta-lactone, 0.4 g, 11.8 mass %) with a viscous material (3.0 g) which was obtained by sufficiently mixing a carbonate (calcium carbonate (also workable as a water-insoluble or poorly soluble calcium salt), 0.6 mass % and sodium bicarbonate 0.4 mass %), a thickener (sodium carboxymethylcellulose, 0.4 mass %), a gelating agent (also workable as a thickener) (sodium alginate, 3.1 mass %), a spreading agent (1,3-butyleneglycol, 6.2 mass %), a preservative (pentyleneglycol, 2.4 mass % and phenoxyethanol, 0.4 mass %) and water (purified water, 74.7 mass %).

### Example 36 Preparation of the composition for preparing a carbon dioxide external preparation [2-2]

The composition for preparing a carbon dioxide external preparation of Example 36 was obtained by combining a substance generating an acid after being hydrolyzed (pantolactone, 1.2 g, 28.6 mass %) with a viscous material (3.0 g) which was obtained by sufficiently mixing a carbonate (calcium carbonate (also workable as a water-insoluble or poorly soluble calcium salt), 0.3 mass %), a thickener (sodium carboxymethylcellulose, 0.6 mass %), a gelating agent (also workable as a thickener) (sodium alginate, 2.5 mass %), a spreading agent (1,3-butyleneglycol, 5.0 mass %), a preservative (pentyleneglycol, 1.9 mass % and phenoxyethanol, 0.4 mass %) and water (purified water, 60.7 mass %).

### Example 37 Preparation of the composition for preparing a carbon dioxide external preparation [2-2]

The composition for preparing a carbon dioxide external preparation of Example 37 was obtained by combining a substance generating an acid after being hydrolyzed (D,L-lactide, 0.3 g, 9.1 mass %) with a viscous material (3.0 g) which was obtained by sufficiently mixing a carbonate (calcium carbonate (also workable as a water-insoluble or poorly soluble calcium salt), 0.6 mass % and sodium bicarbonate, 0.5 mass %), a thickener (sodium carboxymethylcellulose, 0.5 mass %), a gelating agent (also workable as a thickener) (sodium alginate, 3.2 mass %), a spreading agent (1,3-butyleneglycol, 6.4 mass %), a preservative (pentyleneglycol, 2.5 mass % and phenoxyethanol, 0.5 mass %) and water (purified water, 76.7 mass %).

### Example 38 Preparation of the composition for preparing a carbon dioxide external preparation [2-2]

The composition for preparing a carbon dioxide external preparation of Example 38 was obtained by combining a substance generating an acid after being hydrolyzed (D,L-lactide, 0.4 g, 11.8 mass %) with a viscous material (3.0 g) which was obtained by sufficiently mixing a carbonate (calcium carbonate (also workable as a water-insoluble or poorly soluble calcium salt), 0.6 mass % and sodium bicarbonate, 0.4 mass %), a thickener (sodium carboxymethylcellulose, 0.4 mass %), a gelating agent (also workable as a thickener) (sodium alginate, 3.1 mass %), a spreading agent (1,3-butyleneglycol, 6.2 mass %), a preservative (pentyleneglycol, 2.4 mass % and phenoxyethanol, 0.4 mass %) and water (purified water, 74.7 mass %).

### Example 39 Preparation of the composition for preparing a carbon dioxide external preparation [2-2]

The composition for preparing a carbon dioxide external preparation of Example 39 was obtained by combining a substance generating an acid after being hydrolyzed (phthalic anhydride, 0.2 g, 6.3 mass %) with a viscous material (3.0 g) which was obtained by sufficiently mixing a carbonate (calcium carbonate (also workable as a water-insoluble or poorly soluble calcium salt), 0.7 mass % and sodium bicarbonate, 0.5 mass %), a thickener (sodium carboxymethylcellulose, 0.5 mass %), a gelating agent (also workable as a thickener) (sodium alginate, 3.3 mass %), a spreading agent (1,3-butyleneglycol, 6.6 mass %), a preservative (pentyleneglycol, 2.5 mass % and phenoxyethanol, 0.5 mass %) and water (purified water, 79.1 mass %).

### Example 40 Preparation of the composition for preparing a carbon dioxide external preparation [2-2]

The composition for preparing a carbon dioxide external preparation of Example 40 was obtained by combining a substance generating an acid after being hydrolyzed (maleic anhydride, 0.05 g, 1.6 mass %) with a viscous material (3.0 g) which was obtained by sufficiently mixing a carbonate (calcium carbonate (also workable as a water-insoluble or poorly soluble calcium salt), 0.7 mass % and sodium bicarbonate, 0.5 mass %), a thickener (sodium carboxymethylcellulose, 0.5 mass %), a gelating agent (also workable as a thickener) (sodium alginate, 3.4 mass %), a spreading agent (1,3-butyleneglycol, 6.9 mass %), a preservative (pentyleneglycol, 2.7 mass % and phenoxyethanol, 0.5 mass %) and water (purified water, 83.2 mass %).

### Example 41 Preparation of the composition for preparing a carbon dioxide external preparation [2-2]

The composition for preparing a carbon dioxide external preparation of Example 41 was obtained by combining a substance generating an acid after being hydrolyzed (succinic anhydride, 0.1 g, 3.2 mass %) with a viscous material (3.0 g) which was obtained by sufficiently mixing a carbonate (calcium carbonate (also workable as a water-insoluble or poorly soluble calcium salt), 0.7 mass % and sodium bicarbonate, 0.5 mass %), a thickener (sodium carboxymethylcellulose, 0.5 mass %), a gelating agent (also workable as a thickener) (sodium alginate, 3.4 mass %), a spreading agent (1,3-butyleneglycol, 6.8 mass %), a preservative (pentyleneglycol, 2.6 mass % and phenoxyethanol, 0.5 mass %) and water (purified water, 81.8 mass %).

### Example 42 Preparation of the composition for preparing a carbon dioxide external preparation [2-2]

The composition for preparing a carbon dioxide external preparation of Example 42 is obtained by combining a substance generating an acid after being hydrolyzed (D,L-lactide, 2.0 mass % and maleic anhydride, 0.6 mass %) with a viscous material which is obtained by sufficiently mixing a carbonate (calcium carbonate (also workable as a water-insoluble or poorly soluble calcium salt), 0.7 mass % and sodium bicarbonate, 0.5 mass %), a thickener (sodium carboxymethylcellulose, 0.5 mass %), a gelating agent (also workable as a thickener) (sodium alginate, 3.4 mass %), a spreading agent (1,3-butyleneglycol, 6.9 mass %), a preservative (pentyleneglycol, 2.7 mass % and phenoxyethanol, 0.5 mass %) and water (purified water, 82.2 mass %).

### Example 43 Preparation of the composition for preparing a carbon dioxide external preparation [2-2]

The composition for preparing a carbon dioxide external preparation of Example 43 is obtained by combining a substance generating an acid after being hydrolyzed (glucono-delta-lactone, 5.0 mass % and succinic anhydride, 0.5 mass %) with a viscous material which is obtained by sufficiently mixing a carbonate (calcium carbonate (also workable as a water-insoluble or poorly soluble calcium salt), 0.7 mass % and sodium bicarbonate, 0.5 mass %), a thickener (sodium carboxymethylcellulose, 0.5 mass %), a gelating agent (also workable as a thickener) (sodium alginate, 3.4 mass %), a spreading agent (1,3-butyleneglycol, 6.8 mass %), a preservative (pentyleneglycol, 2.6 mass % and phenoxyethanol, 0.5 mass %) and water (purified water, 79.5 mass %).

### Comparative Example 1 Preparation of the composition for preparing a carbon dioxide external preparation wherein a substance generating an acid after being hydrolyzed is not included

### Preparation of the granular material

According to Example 3 described in WO 99/024043, the granular material of Comparative Example 1 (porous columnar granule) was prepared by wet extrusion granulation using water as a solvent, wherein 60 mass % of lactose, 20 mass % of citric acid, 10 mass % of dextrin and 10 mass % of potato starch (2.68, 0.89, 0.45 and 0.45 mass %, respectively, compared to the whole composition) were used.

### Preparation of the viscous composition

According to Example 3 described in WO 99/024043, 3.0 mass % of sodium bicarbonate (2.87 mass % compared to the whole composition); 0.1 mass % of mulberry root extract, panax ginseng root extract, Perilla ocymoides extract, Lithospermum officinale extract and rosemary extract (each 0.10 mass % compared to the whole composition); and 3.0 mass % of 1,2-pentanediol (2.87 mass % compared to the whole composition) were dissolved in 88.50 mass % of a purified water (84.55 mass % compared to the whole composition), and the solution was warmed up to 60°C while 3.0 mass % of sodium alginate (2.87 mass % compared to the whole composition) and 2.0 mass % of sodium carboxymethylcellulose (1.91 mass % compared to the whole composition) were added little by little under stirring and dissolved. The viscous composition of the Comparative Example 1 was then prepared by leaving the solution overnight and cooling it to room temperature.

The composition for preparing a carbon dioxide external preparation of Comparative Example 1 was obtained by combining the granular material and the viscous composition above.

### Comparative Example 2 Preparation of the composition for preparing a carbon dioxide external preparation wherein a substance generating an acid after being hydrolyzed is not included

### Preparation of the granular material

In the same manner as Comparative Example 1, the granular material of Comparative Example 2 (porous columnar granule) was prepared by wet extrusion granulation using water as a solvent, wherein 60 mass % of lactose, 20 mass % of citric acid, 10 mass % of dextrin and 10 mass % of potato starch (2.31, 0.77, 0.38 and 0.38 mass %, respectively, compared to the whole composition) were used.

### Preparation of the viscous composition

0.96 mass % of sodium bicarbonate and 2.88 mass % of 1,2-pentanediol were dissolved in 87.50 mass % of purified water, and thereto were added 2.88 mass % of sodium alginate and 1.92 mass % of sodium carboxymethylcellulose little by little under stirring and the resulted mixture was placed overnight to give the viscous composition of Reference Example 2.

The composition for preparing a carbon dioxide external preparation of Comparative Example 2 was obtained by combining the granular material and the viscous composition described above.

The effects of the present invention are specifically explained by the following test examples.

### Test Example 1 Evaluation of the composition for preparing a carbon dioxide external preparation [1-1]

Carbon dioxide external preparations obtained from the compositions for preparing a carbon dioxide external preparation of Example 1 to 6 were applied to the left forearm of a female subject of 28-years old, and visually evaluated about the evaluation items shown below. Comprehensive evaluation was performed from the results, which are shown in Table 1.

### Solubility of the granular material

Evaluations: ⊚: easily soluble, ○: soluble, Δ: slightly soluble, ×: insoluble

### Drooping of the preparation

Evaluations: ⊚: no drooping, Δ: slightly drooping, ×: drooping

### Reaction of the skin absorbing carbon dioxide (reaction such as redness or whitening of the applied site)

Evaluations: ⊚: remarkably reacted, ○: moderately reacted, Δ: slightly reacted, ×: no reaction

### Comprehensive evaluation

Very Good: ⊚ in every item
Good: ⊚ or ○ in Reaction of the skin absorbing carbon dioxide and ⊚ in one of other items at least
Not Good: × in Reaction of the skin absorbing carbon dioxide

**Table 1**

| Example No. | 1 | 2 | 3 | 4 | 5 | 6 |
|---|---|---|---|---|---|---|
| The amount of external preparation (g) | 5.0 | 5.0 | 5.0 | 5.0 | 5.0 | 0.7 |
| Applied area (cm²) | 25 | 25 | 25 | 25 | 25 | 9 |
| Solubility or dispersibility of the granular material | ⊚ | ○ | ○ | ⊚ | ⊚ | ⊚ |
| Drooping of the carbon dioxide external preparation | ⊚ | ⊚ | ⊚ | ⊚ | ⊚ | ⊚ |
| Reaction of the skin absorbing carbon dioxide | ⊚ | ⊚ | ⊚ | ⊚ | ⊚ | ○ |
| Comprehensive evaluation | Very Good | Good | Good | Very Good | Very Good | Good |

### Test Example 2 Evaluation of the composition for preparing a carbon dioxide external preparation [1-2]

Carbon dioxide external preparations obtained from the compositions for preparing a carbon dioxide external preparation of Example 7 and 8 were evaluated in the same manner as Test Example 1 except that the subject was replaced with a female of 39 years old and state of the application below was added as an additional item for evaluation.

### State of the application

Evaluation:
   ⊚: Applicable so that surface of the applied site is smooth,
   ○: Applicable so that surface of the applied site is slightly smooth,
   Δ: Applicable but surface of the applied site is irregular,
   ×: Not applicable

**Table 2**

| Example No. | 7 | 8 |
|---|---|---|
| The amount of external preparation (g) | 3.0 | 3.0 |
| Applied area (cm²) | 25 | 25 |
| Solubility or dispersibility of the granular material | ⊚ | ⊚ |
| State of the application | ⊚ | ⊚ |
| Drooping of the carbon dioxide external preparation | ⊚ | ⊚ |
| Reaction of the skin absorbing carbon dioxide | ⊚ | ⊚ |
| Comprehensive evaluation | Very Good | Very Good |

### Test Example 3 Evaluation of the composition for preparing a carbon dioxide external preparation [2-1]

Carbon dioxide external preparations obtained from the compositions for preparing a carbon dioxide external preparation of Example 9 to 11 were applied to the left forearm of a female subject of 30 years old, and visual and tactual evaluation on the next items was conducted. Further, comprehensive evaluation was performed from the results, which are shown in Table 3.

### Solubility or dispersibility of the granular material

Evaluation:
   ⊚: Almost completely soluble or dispersable,
   ○: Over half of the material is soluble or dispersable,
   Δ: Less than quarter is soluble or dispersable,
   ×: Almost insoluble or undispersable;

### State of the application

Evaluation:
   ⊚: Applicable so that surface of the applied site is smooth,
   ○: Applicable so that surface of the applied site is slightly smooth,
   Δ: Applicable but surface of the applied site is irregular,
   ×: Not applicable

### Reaction of the skin absorbing carbon dioxide (reaction such as redness or whitening of the applied site)

Evaluation: ⊚: remarkably reacted, ○: moderately reacted, Δ: slightly reacted, ×: no reaction

### State of the gelation 30 minutes later

Evaluation: ⊚: gelated as a whole, ○: gelated only on the surface, ×: Not gelated

### Peeling-off 30 minutes later

Evaluation:
   ⊚: Can be peeled-off easily,
   ○: can be peeled-off in more than one sheet,
   Δ: can be peeled-off but is sticky paste or fragile sheet;
   ×: Cannot be peeled-off

### Comprehensive evaluation

Very Good: ⊚ in every item
Good: ⊚ or ○ in Reaction of the skin absorbing carbon dioxide and ⊚ in one of other items at least
Not Good: × in Reaction of the skin absorbing carbon dioxide

**Table 3**

| Example No. | | 9 | 10 | 11 |
|---|---|---|---|---|
| The amount of external preparation (g) | A | 0.8 | 1.3 | 0.55 |
| | B | 0.8 | 1.3 | 0.55 |
| Applied area (cm²) | A | 9 | 9 | 9 |

| | B | 9 | 9 | 9 |
|---|---|---|---|---|
| Solubility or dispersibility of the granular material | | ⊚ | Δ | ⊚ |
| State of the application | A | ○ | ⊚ | ⊚ |
| | B | Δ | Δ | - |
| Reaction of the skin absorbing carbon dioxide | | ○ | ⊚ | ⊚ |
| State of the gelation 30 minutes later | A | ⊚ | ⊚ | ⊚ |
| | B | ○ | ⊚ | - |
| Peeling 30 minutes later | A | ⊚ | ⊚ | ⊚ |
| | B | ○ | Δ | - |
| Comprehensive evaluation | | Good | Good | Very Good |

| | | | | |
|---|---|---|---|---|
| A: applied immediately after being mixed B: applied 5 minutes after being mixed | | | | |

### Test Example 4 Evaluation of the hydrogel, the composition for preparing a carbon dioxide external preparation [2-1]

The composition for preparing a carbon dioxide external preparation of Example 12 was moistened with water and applied to the forearm of a female subject of 30-years old. The skin of the applied site showed redness five minutes later, and a vasodilating effect was clearly observed. The surface of unwoven fabric was wet when touched with fingers. Ten minutes later, hydrogel was formed on the surface and the fingers did not get wet.

### Test Example 5 Treatment of a wound, the composition for preparing a carbon dioxide external preparation [2-1]

The composition for preparing a carbon dioxide external preparation of Example 12 was moistened with water and applied to a small cut of the right forefinger of a female of 28-years old. Twenty minutes later, the cut was completely closed. Test Example 6 Evaluation of the composition for preparing a carbon dioxide external preparation [2-2]

Carbon dioxide external preparations obtained from the compositions for preparing a carbon dioxide external preparation of Example 13 to 25 were evaluated in the same manner as Test Example 3, and the results are shown in Table 4 and 5.

**Table 4**

| Example No. | | 13 | 14 | 15 | 16 | 17 | 18 | 19 |
|---|---|---|---|---|---|---|---|---|
| The amount of external preparation (g) | A | 1.5 | 1.5 | 1.5 | 1.5 | 1.5 | 1.5 | 1.5 |
| | B | 1.5 | 1.5 | 1.5 | 1.5 | 1.5 | 1.5 | 1.5 |
| Applied area (cm²) | A | 9 | 9 | 9 | 9 | 9 | 9 | 9 |
| | B | 9 | 9 | 9 | 9 | 9 | 9 | 9 |
| Solubility or dispersibility of the granular material | | Δ | Δ | ⊚ | ⊚ | ⊚ | Δ | Δ |
| State of the application | A | Δ | Δ | ⊚ | ⊚ | ⊚ | ○ | ⊚ |
| | B | Δ | Δ | ⊚ | ⊚ | ⊚ | ○ | ○ |
| Reaction of the skin absorbing carbon dioxide | | ○ | ○ | ⊚ | ⊚ | ⊚ | ⊚ | ⊚ |
| State of the gelation 30 minutes later | A | ⊚ | ⊚ | ⊚ | ⊚ | ⊚ | ⊚ | ⊚ |
| | B | ⊚ | ⊚ | ⊚ | ⊚ | ⊚ | ⊚ | ⊚ |
| Peeling-off 30 minutes later | A | Δ | Δ | Δ | ⊚ | ○ | Δ | ⊚ |
| | B | Δ | Δ | Δ | ⊚ | ○ | Δ | ○ |
| Comprehensive evaluation | | Good | Good | Good | Very Good | Good | Good | Good |

| | | | | | | | | |
|---|---|---|---|---|---|---|---|---|
| A: applied immediately after being mixed B: applied 5 minutes after being mixed | | | | | | | | |

**Table 5**

| Example No. | | 20 | 21 | 22 | 23 | 24 | 25 |
|---|---|---|---|---|---|---|---|
| The amount of external preparation (g) | A | 1.5 | 1.5 | 1.5 | 1.5 | 1.5 | 1.5 |
| | B | 1.5 | 1.5 | 1.5 | 1.5 | 1.5 | 1.5 |
| Applied area (cm²) | A | 9 | 9 | 9 | 9 | 9 | 9 |
| | B | 9 | 9 | 9 | 9 | 9 | 9 |
| Solubility or dispersibility of the granular material | | ⊚ | ⊚ | ⊚ | ⊚ | Δ | Δ |
| State of the application | A | ⊚ | ⊚ | ⊚ | ⊚ | ⊚ | ⊚ |
| | B | ⊚ | ⊚ | ⊚ | ⊚ | Δ | ○ |
| Reaction of the skin absorbing carbon dioxide | | ⊚ | ⊚ | ⊚ | ⊚ | ⊚ | ⊚ |
| State of the gelation 30 minutes later | A | ○ | ⊚ | ⊚ | ⊚ | ⊚ | ⊚ |
| | B | ○ | ⊚ | ⊚ | ⊚ | ⊚ | ⊚ |
| Peeling-off 30 minutes later | A | Δ | ⊚ | Δ | Δ | ○ | ○ |
| | B | Δ | ⊚ | Δ | Δ | Δ | ○ |
| Comprehensive evaluation | | Good | Very Good | Good | Good | Good | Good |

| | | | | | | | |
|---|---|---|---|---|---|---|---|
| A: applied immediately after being mixed B: applied 5 minutes after being mixed | | | | | | | |

### Test Example 7 Example of evaluating cosmetic effects

Each 26.6 g of the carbon dioxide external preparation obtained from the composition for preparing a carbon dioxide external preparation of Example 26 was applied to the face of a female subject of 28-years old, or 41-years old or a male subject of 49-years old.

The carbon dioxide external preparation showed such good spreadability that it could be applied to the overall face in a uniform thickness and never drooped. After being applied, the preparation delivered an extremely notable cooling sensation and caused skin redness, which were sustained for about ten minutes. After 30 minutes, the carbon dioxide external preparation solidified to form a hydrogel sheet and was readily peeled off from the face. It was observed that the carbon dioxide external preparation made the skins of all the subjects more white and smoother.

### Test Example 8 Evaluation of Example 27

Each 13.30 g of the carbon dioxide external preparation prepared from the composition for preparing a carbon dioxide external preparation of Example 27 was applied to the half face of the female subject of 28-years, 30-years or 41-years old. The carbon dioxide external preparation showed good spreadability and could be applied in a uniform thickness on the whole half face, and never drooped.

On the other hand, 13.45 g of the carbon dioxide external preparation was prepared from the composition for preparing a carbon dioxide external preparation of Comparative Example 1 (0.60 g of the granular material and 12.85 g of the viscous material were mixed), and applied to the other half face of each subject.

After 30 minutes, the carbon dioxide external preparations of Example 27 and Comparative Example 1 were both removed completely from the face of each subject. The preparation of Example 27 solidified like a hydrogel sheet and was readily peeled off from the face and no facial washing was required. On the contrary, the preparation of Comparative Example 1 was highly viscous fluid and the subjects had to wash their faces for several minutes to completely remove the preparations. Compared to the half face which Comparative Example 1 was applied, the half face which Example 27 was applied was superior in terms of feeling of skin moisture, sagging skin, effect of making a face look smaller, whiteness, brightness, clearness, softness and fine-texture of skin in every subject.

### Test Example 9 Comparative test when the same amount of the carbon dioxide external preparation was applied

3.14 g of the carbon dioxide external preparations (hereinafter, the example external preparation 1A) was prepared from the composition for preparing a carbon dioxide external preparation of Example 1, and 3.14 g of the carbon dioxide external preparations was prepared from the composition for preparing a carbon dioxide external preparation of Comparative Example 1 (0.14 g of the granular material and 3 g of the viscous composition were mixed; hereinafter the comparative example external preparation 1A). The two carbon dioxide external preparations obtained were applied to the left forearm of the female subject of 41-years old 3 cm away from each other, with the same thickness and area. After 3 minutes, the applied sites of the skin showed redness in both of the example external preparation 1A and the comparative example external preparation 1A. But, the example external preparation 1A showed slightly intensive redness. Further 5, 10, 15, 20 and 30 minutes later, the applied sites of the skin showed redness in both carbon dioxide external preparations, and in all cases the example external preparation 1A showed a little more intensive redness.

### Test Example 10 Comparative test when the same amount of the carbon dioxide is (theoretically) generated using the composition for preparing a carbon dioxide external preparation [1-1]

1.50 g of the carbon dioxide external preparation (hereinafter, the example external preparation 4B; 7.86 mg of the theoretical amount of carbon dioxide was generated) was prepared from the composition for preparing a carbon dioxide external preparation of Example 4, and 1.56 g of the carbon dioxide external preparations (hereinafter, the comparative example external preparation 2B; 7.86 mg of the theoretical amount of carbon dioxide was generated) was prepared from the composition for preparing a carbon dioxide external preparation of Comparative Example 2 (0.06 g of the granular material and 1.50 g of the viscous material).

The two carbon dioxide external preparations obtained above were applied to the right femoral of a female subject of 29-years old 3 cm away from each other, with the same thickness. 3 minutes later, the skin of the applied sites showed redness in both of the example external preparation 4B and the comparative example external preparation 2B. However, the example external preparation 4B showed remarkably more intensive redness than that of the comparative example external preparation 2B after five minutes have passed, in every time points of 10 minutes, 15 minutes, 20 minutes and 30 minutes later.

The same amount of carbon dioxide is thought to be theoretically generated in the example external preparation 4B and the comparative example external preparation 2B since they both have 0.015 g of sodium bicarbonate, and enough amount of glucono-delta-lactone or an acid to react with the whole sodium bicarbonate. This evaluation showed that the composition for preparing a carbon dioxide external preparation of the present invention provides the carbon dioxide external preparation, wherein the amount of transdermally absorbed carbon dioxide is increased and sustained longer despite the amount of carbon dioxide-source is the same compared to the prior art.

### Test Example 11 Comparative test when the same amount of the carbon dioxide is generated (theoretically) using the composition for preparing a carbon dioxide external preparation [1-2]

1.57 g of the carbon dioxide external preparation (hereinafter, the example external preparation 28C; 7.86 mg of the theoretical amount of carbon dioxide generated) was prepared from the composition for preparing a carbon dioxide external preparation of Example 28. Further, 1.56 g of the comparative example external preparation 2B (7.86 mg of the theoretical amount of carbon dioxide generated) was prepared. The two carbon dioxide external preparations obtained were applied to the right femoral region of a female subject of 29-years old 3 cm away from each other, with the same thickness. 3 minutes later, the skin of the applied sites showed redness in both of the example external preparation 28C and the comparative example external preparation 2B. However, the example external preparation 28C showed remarkably more intensive redness than that of the comparative example external preparation 2B whenever observed after five minutes, in the same manner as Test Example 10 above. Test Example 12 Evaluation of the composition for preparing a carbon dioxide external preparation [2-2]

The carbon dioxide external preparations obtained from the composition for preparing a carbon dioxide external preparation of Example 35 to 41 were evaluated in the same manner as Test Example 3 described before. The results are shown in Table 6.

**Table 6**

| Example No. | | 35 | 36 | 37 | 38 | 39 | 40 | 41 |
|---|---|---|---|---|---|---|---|---|
| The amount of external preparation (g) | A | 1.5 | 1.5 | 1.5 | 1.5 | 1.5 | 1.5 | 1.5 |
| | B | 1.5 | 1.5 | 1.5 | 1.5 | 1.5 | 1.5 | 1.5 |
| Applied area (cm²) | A | 9 | 9 | 9 | 9 | 9 | 9 | 9 |
| | B | 9 | 9 | 9 | 9 | 9 | 9 | 9 |
| Solubility or dispersibility of the granular material | | ⊚ | ⊚ | ○ | ○ | ○ | ○ | ○ |
| State of the application | A | ⊚ | ⊚ | ⊚ | ⊚ | ⊚ | ○ | ⊚ |
| | B | ○ | ⊚ | ○ | ⊚ | ⊚ | × | × |
| Reaction of the skin absorbing carbon dioxide | | ⊚ | ○ | ⊚ | ⊚ | ○ | ○ | ○ |
| State of the gelation 30 minutes later | A | ⊚ | ⊚ | ⊚ | ⊚ | ⊚ | ○ | ○ |
| | B | ⊚ | ⊚ | ⊚ | ⊚ | ⊚ | 1) | 1) |
| Peeling-off 30 minutes later | A | ⊚ | ⊚ | ⊚ | ⊚ | ⊚ | ⊚ | ⊚ |
| | B | ⊚ | ⊚ | ⊚ | ⊚ | ⊚ | 1) | 1) |
| Comprehensive evaluation | | Good | Good | Good | Good | Good | Good | Good |

| | | | | | | | | |
|---|---|---|---|---|---|---|---|---|
| A: applied immediately after being mixed B: applied 5 minutes after being mixed 1) Preparations could not be applied since hydrogelation was too fast. | | | | | | | | |

## Claims

1. A composition for preparing a carbon dioxide external preparation for skin and mucosa comprising; a substance generating an acid after being hydrolyzed, a carbonate, a thickener, water, a gelating agent being gelated by calcium ion, and a water-insoluble or poorly soluble calcium salt as essential components, which are provided in a form consisting of a component (a) which is a granular material, and a component (b) which is water or a viscous material,
wherein
(i) when (b) is water the substance generating an acid after being hydrolyzed, the carbonate, the thickener, the gelating agent being gelated by calcium ion, and the water-insoluble or poorly soluble calcium salt are provided in the component (a) granular material which is to be mixed with the component (b) water just before use; or
(ii) when (b) is a viscous material the substance generating an acid after being hydrolyzed is provided in the component (a) granular material, which is to be mixed with the component (b) viscous material comprising the thickener and the water, and wherein the carbonate, the gelating agent being gelated by calcium ion, and the water-insoluble or poorly soluble calcium salt are contained either in the component (a) granular material or in the component (b) viscous material,
wherein the substance generating an acid after being hydrolyzed is one or more selected from glucono-delta-lactone, pantolactone, D,L- or L-lactide (3,6-dimethyl-1,4-dioxane-2,5-dione), D,L- or L-glycolide, phthalic anhydride, maleic anhydride, and succinic anhydride; and
wherein the mixing ratios of the substance generating an acid after being hydrolyzed is 0.5 to 40 mass %, of the carbonate is 0.1 to 10 mass %, of the thickener is 0.5 to 10 mass %, of the water is 40 to 95 mass %, of the gelating agent is 0.5 to 10 mass %, and the water-insoluble or poorly soluble calcium salt is 0.1 to 5 mass %, relative to the total amount of the composition.

2. The composition according to claim 1,
wherein the substance generating an acid after being hydrolyzed is provided in the component (a) granular material, which is to be mixed with the component (b) viscous material comprising the thickener, the water, the carbonate, the gelating agent being gelated by calcium ion, and the water-insoluble or poorly soluble calcium salt just before use.

3. The composition according to any one of the preceding claims,
wherein the substance generating an acid after being hydrolyzed, the carbonate, the thickener, the gelating agent being gelated by calcium ion, and the water-insoluble or poorly soluble calcium salt are provided in a form of a granular material which is to be mixed with water just before use; and
the granular material further contains a dispersant, which is one or more selected from pregelatinized starch, α-cyclodextrin, sugar, glucose, fructose, sucrose, lactose, xylitol, D-sorbitol, D-mannitol; pullulan, xanthan gum, hydroxypropylcellulose, hydroxypropylmethylcellulose, carmellose calcium, carmellose sodium, polyvinyl pyrrolidone and urea.

4. The composition according to any one of the preceding claims,
wherein the carbonate is one or more selected from ammonium carbonate, ammonium bicarbonate, potassium carbonate, potassium bicarbonate, potassium sesquicarbonate, sodium carbonate, sodium bicarbonate, sodium sesquicarbonate, lithium carbonate, lithium bicarbonate, lithium sesquicarbonate, cesium carbonate, cesium bicarbonate, cesium sesquicarbonate, magnesium carbonate, magnesium bicarbonate, calcium bicarbonate, calcium carbonate, magnesium hydroxide carbonate, and barium carbonate;
the water-insoluble or poorly soluble calcium salt is one or more selected from calcium carbonate, calcium sulfate, calcium citrate, calcium alginate, calcium gluconate, calcium pyrophosphate, monobasic calcium phosphate and calcium silicate; and
the gelating agent being gelated by calcium ion is one or more selected from sodium alginate, carrageenan, tara gum and locust bean gum.

5. The composition according to any one of the preceding claims,
wherein the thickener and the gelating agent being gelated by calcium ion is sodium alginate.

6. The composition according to any one of the preceding claims,
wherein the carbonate and the water-insoluble or poorly soluble calcium salt is calcium carbonate.

7. A material for use in treating a wound or burn comprising a carbon dioxide external agent for skin and mucosa obtained from the composition for preparing a carbon dioxide external preparation for skin and mucosa according to any one of the preceding claims.

## Patentansprüche

1. Zusammensetzung für die Herstellung eines äußerlichen Kohlendioxidpräparats für Haut und Schleimhäute,
die Folgendes aufweist:
eine Substanz, die eine Säure erzeugt, nachdem sie hydrolysiert worden ist, ein Carbonat, ein Verdickungsmittel, Wasser, ein Geliermittel, das durch Calciumionen geliert, und ein in Wasser unlösliches oder schwer lösliches Calciumsalz als wesentliche Bestandteile, die in einer Form bereitgestellt werden, die aus einer Komponente (a), die ein körniges Material ist, und einer Komponente (b) besteht, die Wasser oder ein viskoses Material ist,
wobei
(i) wenn (b) Wasser ist, die Substanz, die eine Säure erzeugt, nachdem sie hydrolysiert worden ist, das Carbonat, das Verdickungsmittel, das Geliermittel, das durch Calciumionen geliert, und das in Wasser unlösliche oder schwer lösliche Calciumsalz in der Komponente in Form eines körnigen Materials (a) bereitgestellt werden, die direkt vor der Anwendung mit der Komponente (b) gemischt werden muß; oder
(ii) wenn (b) ein viskoses Material ist, die Substanz, die eine Säure erzeugt, nachdem sie hydrolysiert worden ist, in der Komponente in der Form eines körnigen Materials (a) bereitgestellt wird, die mit der Komponente in Form eines viskosen Materials (b) gemischt werden muß, die das Verdickungsmittel und das Wasser aufweist, und wobei das Carbonat, das Geliermittel, das durch Calciumionen geliert, und das in Wasser unlösliche oder schwer lösliche Calciumsalz entweder in der Komponente in Form eines körnigen Materials (a) oder in der Komponente in Form eines viskosen Materials (b) enthalten sind,
wobei die Substanz, die eine Säure erzeugt, nachdem sie hydrolysiert worden ist, eine oder mehrere ist, die aus Glucono-δ-lacton, Pantolacton, D,L- oder L-Lactid (3,6-Dimethyl-1,4-dioxan-2,5-dion), D,L- oder L-Glycolid, Phthalsäureanhydrid, Maleinsäureanhydrid und Succinsäureanhydrid ausgewählt sind; und
wobei die Mischungsanteile der Substanz, die eine Säure erzeugt, nachdem sie hydrolysiert worden ist, 0,5 bis 40 Massen-%, des Carbonats 0,1 bis 10 Massen-%, des Verdickungsmittels 0,5 bis 10 Massen-%, des Wassers 40 bis 95 Massen-%, des Geliermittels 0,5 bis 10 Massen-% und des in Wasser unlöslichen oder schwer löslichen Calciumsalzes 0,1 bis 5 Massen-% betragen, und zwar auf die Gesamtmenge der Zusammensetzung bezogen.

2. Zusammensetzung nach Anspruch 1,
wobei die Substanz, die eine Säure erzeugt, nachdem sie hydrolysiert worden ist, in der Komponente in Form eines körnigen Materials (a) bereitgestellt wird, die direkt vor der Anwendung mit der Komponente in Form eines viskosen Materials (b) gemischt werden muß, die das Verdickungsmittel, das Wasser, das Carbonat, das Geliermittel, das durch Calciumionen geliert, und das in Wasser unlösliche oder schwer lösliche Calciumsalz aufweist.

3. Zusammensetzung nach einem der vorstehenden Ansprüche,
wobei die Substanz, die eine Säure erzeugt, nachdem sie hydrolysiert worden ist, das Carbonat, das Verdickungsmittel, das Geliermittel, das durch Calciumionen geliert, und das in Wasser unlösliche oder schwer lösliche Calciumsalz in Form eines körnigen Materials bereitgestellt werden, das direkt vor der Anwendung mit Wasser gemischt werden muß; und
wobei das körnige Material ferner ein Dispersionsmittel enthält, das eines oder mehrere ist, die aus vorgelatinierter Stärke, α-Cyclodextrin, Zucker, Glucose, Fructose, Saccharose, Lactose, Xylitol, D-Sorbitol, D-Manitol; Pullulan, Xanthangummi, Hydroxypropylcellulose, Hydroxypropylmethylcellulose, Carmellose-Calcium, Carmellose-Natrium, Polyvinylpyrrolidin und Harnstoff ausgewählt sind.

4. Zusammensetzung nach einem der vorstehenden Ansprüche,
wobei das Carbonat eines oder mehrere ist, die ausgewählt sind aus Ammoniumcarbonat, Ammoniumbicarbonat, Kaliumcarbonat, Kaliumbicarbonat, Kaliumsesquicarbonat, Natriumcarbonat, Natriumbicarbonat, Natriumsesquicarbonat, Lithiumcarbonat, Lithiumbicarbonat, Lithiumsesquicarbonat, Cäsiumcarbonat, Cäsiumbicarbonat, Cäsiumsesquicarbonat, Magnesiumcarbonat, Magnesiumbicarbonat, Calciumbicarbonat, Calciumcarbonat, Magnesiumhydroxidcarbonat und Bariumcarbonat;
wobei das in Wasser unlösliche oder schwer lösliches Calciumsalz eines oder mehrere ist, die aus Calciumcarbonat, Calciumsulfat, Calciumcitrat, Calciumalginat, Calciumgluconat, Calciumpyrophosphat, einwertigem Calciumphosphat und Calciumsilicat ausgewählt sind; und
wobei das Geliermittel, das durch Calciumionen geliert, eines oder mehrere ist, die aus Natriumalginat, Carrageen, Tarakernmehl und Johannisbrotkernmehl ausgewählt sind.

5. Zusammensetzung nach einem der vorstehenden Ansprüche,
wobei das Verdickungsmittel und das Geliermittel, das durch Calciumionen geliert, Natriumalginat sind.

6. Zusammensetzung nach einem der vorstehenden Ansprüche,
wobei das Carbonat und das in Wasser unlösliche oder schwer lösliche Calciumsalz Calciumcarbonat sind.

7. Material für die Verwendung bei der Behandlung von Wunden oder Verbrennungen,
das ein äußerliches Kohlendioxidmittel für Haut und Schleimhäute aufweist, das aus der Zusammensetzung für die Herstellung eines äußerlichen Kohlendioxidpräparats für Haut und Schleimhäute nach einem der vorstehenden Ansprüche erhalten worden ist.

## Revendications

1. Composition pour la préparation d'une préparation externe de dioxyde de carbone pour la peau et les muqueuses comprenant :
une substance générant un acide après avoir été hydrolysée, un carbonate, un épaississant, de l'eau, un agent gélifiant étant gélifié par l'ion calcium, et un sel de calcium insoluble ou médiocrement soluble dans l'eau comme constituants essentiels, lesquels sont fournis dans une forme constituée d'un constituant (a) qui est un matériau de granulé, et d'un constituant (b) qui est l'eau ou un matériau visqueux,
dans laquelle
(i) lorsque (b) est l'eau la substance générant un acide après avoir été hydrolysée, le carbonate, l'épaississant, l'agent gélifiant étant gélifié par l'ion calcium, et le sel de calcium insoluble ou médiocrement soluble dans l'eau sont fournis dans le matériau de granulé de constituant (a) qui doit être mélangé avec l'eau de constituant (b) juste avant l'utilisation ; où
(ii) lorsque (b) est un matériau visqueux, la substance générant un acide après avoir été hydrolysée est fournie dans le matériau de granulé de constituant (a), lequel doit être mélangé avec le matériau visqueux de constituant (b) comprenant l'épaississant et l'eau, et dans laquelle le carbonate, l'agent gélifiant étant gélifié par l'ion calcium, et le sel de calcium insoluble ou médiocrement soluble dans l'eau sont contenus soit dans le matériau de granulé de constituant (a) soit dans le matériau visqueux de constituant (b),
dans laquelle la substance générant un acide après avoir été hydrolysée est une ou plusieurs choisies parmi la glucono-delta-lactone, la pantolactone, le D,L- ou L-lactide (3,6-diméthyl-1,4-dioxane-2,5-dione), le D,L- ou L-glycolide, l'anhydride phtalique, l'anhydride maléique, et l'anhydride succinique ; et
dans laquelle les rapports de mélange de la substance générant un acide après avoir été hydrolysée est de 0,5 à 40 % en masse, du carbonate est de 0,1 à 10 % en masse, de l'épaississant est de 0,5 à 10 % en masse, de l'eau est de 40 à 95 % en masse, de l'agent gélifiant est de 0,5 à 10 % en masse, et du sel de calcium insoluble ou médiocrement soluble dans l'eau est de 0,1 à 5 % en masse, par rapport à la quantité totale de la composition.

2. Composition selon la revendication 1,
dans laquelle la substance générant un acide après avoir été hydrolysée est fournie dans le matériau de granulé de constituant (a), qui doit être mélangé avec le matériau visqueux de constituant (b) comprenant l'épaississant, l'eau, le carbonate, l'agent gélifiant étant gélifié par l'ion calcium, et le sel de calcium insoluble ou médiocrement soluble dans l'eau juste avant l'utilisation.

3. Composition selon l'une quelconque des revendications précédentes, dans laquelle la substance générant un acide après avoir été hydrolysée, le carbonate, l'épaississant, l'agent gélifiant étant gélifié par l'ion calcium, et le sel de calcium insoluble ou médiocrement soluble dans l'eau sont fournis dans une forme d'un matériau de granulé qui doit être mélangé avec l'eau juste avant l'utilisation ; et
le matériau de granulé contient de plus un dispersant, lequel est un ou plusieurs choisis parmi un amidon prégélatinisé, l'α-cyclodextrine, un sucre, le glucose, le fructose, le saccharose, le lactose, le xylitol, le D-sorbitol, le D-mannitol ; le pullulane, la gomme xanthane, l'hydroxypropylcelllose, l'hydroxypropylméthylcellulose, le carmellose calcium, le carmellose sodium, la polyvinylpyrrolidone et l'urée.

4. Composition selon l'une quelconque des revendications précédentes, dans laquelle le carbonate est un ou plusieurs choisis parmi le carbonate d'ammonium, le bicarbonate d'ammonium, le carbonate de potassium, le bicarbonate de potassium, le sesquicarbonate de potassium, le carbonate de sodium, le bicarbonate de sodium, le sesquicarbonate de sodium, le carbonate de lithium, le bicarbonate de lithium, le sesquicarbonate de lithium, le carbonate de césium, le bicarbonate de césium, le sesquicarbonate de césium, le carbonate de magnésium, le bicarbonate de magnésium, le bicarbonate de calcium, le carbonate de calcium, le carbonate d'hydroxyde de magnésium, et le carbonate de baryum ;
le sel de calcium insoluble ou médiocrement soluble dans l'eau est un ou plusieurs choisis parmi le carbonate de calcium, le sulfate de calcium, le citrate de calcium, l'alginate de calcium, le gluconate de calcium, le pyrophosphate de calcium, le phosphate de calcium monobasique et le silicate de calcium ; et
l'agent gélifiant étant gélifié par l'ion calcium est un ou plusieurs choisis parmi l'alginate de sodium, le carragheen, la gomme de tara et la gomme de caroube.

5. Composition selon l'une quelconque des revendications précédentes,
dans laquelle l'épaississant et l'agent gélifiant étant gélifié par l'ion calcium est l'alginate de sodium.

6. Composition selon l'une quelconque des revendications précédentes,
dans laquelle le carbonate et le sel de calcium insoluble ou médiocrement soluble dans l'eau est le carbonate de calcium.

7. Matériau destiné à une utilisation dans le traitement d'une plaie ou d'une brûlure comprenant un agent externe de dioxyde de carbone pour la peau et les muqueuses obtenu à partir de la composition pour la préparation d'une préparation externe de dioxyde de carbone pour la peau et les muqueuses selon l'une quelconque des revendications précédentes.
